# EUROPÄISCHE PATENTSCHRIFT

(11) **EP 2 062 977 B1**
(45) Veröffentlichungstag und Bekanntmachung des Hinweises auf die Patenterteilung: **08.01.2014**
(21) Anmeldenummer: 09154489.0
(22) Anmeldetag: 17.02.2000
(51) Int. Cl.: C12P 21/00, C12N 15/54, C12N 9/10

(54) **Fucosyltransferase-Gen**
Fucosyltransferase-gene
Gène de fucosyletransférase

(30) Priorität: 18.02.1999 AT 27099
(43) Veröffentlichungstag der Anmeldung: 27.05.2009
(62) Teilanmeldung aus: 05108012.5
(73) Patentinhaber: Altmann, Friedrich, 1190 Wien (AT)
(72) Erfinder: Altmann, Friedrich, 1190 Wien (AT)
(74) Vertreter: Sonn & Partner Patentanwälte

(56) Entgegenhaltungen:
- EP-B- 1 151 109
- CHRISPEELS M.J. ET AL.: "The production of recombinant glycoproteins with defined non-immunogenic glycans" TRANSGENIC PLANTS: A PRODUCTION SYSTEM FOR INDUSTRIAL AND PHARMACEUTICAL PROTEINS, 1996, Seiten 99-113, XP002940545
- STAUDACHER E ET AL: "Functional purification and characterization of a GDP-fucose: beta-N-acetylglucosamine (Fuc to Asn linked GlcNAc) alpha-1,3-fucosyltransferase from mung beans" GLYCOCONJUGATE JOURNAL, CHAPMAN & HALL, GB, Bd. 12, 1. Januar 1995 (1995-01-01), Seiten 780-786, XP002140246 ISSN: 0282-0080
- SCHAEWEN VON A. ET AL.: "Isolation of a mutant Arabidopsis plant that lacks N-Acetyl Glucosaminyl Transferase I and is unable to synthesize Golgi-modified complex N-linked glycans" PLANT PHYSIOLOGY, AMERICAN SOCIETY OF PLANT PHYSIOLOGISTS, ROCKVILLE, MD, US, Bd. 102, Nr. 4, 1. August 1993 (1993-08-01), Seiten 1109-1118, XP000645269 ISSN: 0032-0889
- LEROUGE P. ET AL.: "N-glycoprotein biosynthesis in plants: recent developments and future trends" PLANT MOLECULAR BIOLOGY, SPRINGER, DORDRECHT, NL, Bd. 38, 1998, Seiten 31-48, XP002140796 ISSN: 0167-4412
- LEITER H ET AL: "Purification, cDNA cloning and expression of GDP-L-Fuc:Asn-linked GlcNAc alpha-1,3-Fucosyltransferase from mung bean" JOURNAL OF BIOLOGICAL CHEMISTRY, AMERICAN SOCIETY OF BIOLOCHEMICAL BIOLOGISTS, BIRMINGHAM, US, Bd. 274, Nr. 31, 30. Juli 1999 (1999-07-30), Seiten 21830-21839, XP002140245 ISSN: 0021-9258
- COSTACHE M ET AL: "EVOLUTION OF FUCOSYLTRANSFERASE GENES IN VERTEBRATES", JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.272.47.29721, vol. 272, no. 47, 21 November 1997 (1997-11-21), pages 29721-29728, XP002925163, ISSN: 0021-9258

## Beschreibung

Die Erfindung betrifft Polynukleotide, die für eine Fucosyltransferase codieren. Weiters betrifft die Erfindung Teilsequenzen dieser Polynukleotide sowie Vektoren mit diesen Polynukleotiden, rekombinante Wirtszellen, Pflanzen und Insekten, die mit den Polynukleotiden bzw. davon abstammender DNA transfektiert sind sowie die in diesen Systemen produzierten Glykoproteine.

Glykoproteine weisen eine Vielfalt und Komplexität von Kohlenhydrat-Einheiten auf, wobei die Zusammensetzung und Anordnung der Kohlenhydrate für unterschiedliche Organismen charakteristisch ist. Die Oligosaccharid-Einheiten der Glykoproteine haben eine Reihe von Aufgaben, so sind sie z.B. wichtig in der Stoffwechselregulation, sie sind an der Vermittlung von Zell-Zell-Wechselwirkungen beteiligt, sie bestimmen die Zirkulationszeiten von Proteinen im Blutkreislauf und sie sind ausschlaggebend für die Erkennung von Epitopen in Antigen-Antikörper-Reaktionen.

Die Glykosylierung von Glykoproteinen beginnt im endoplasmatischen Retikulum (ER), wo die Oligosaccharide entweder durch N-glykosidische Bindungen an Asparagin-Seitenketten oder durch O-glykosidische Bindungen an Serin- oder Threonin-Seitenketten gebunden werden. Die N-gebundenen Oligosaccharide enthalten ein gemeinsames Core aus einer Pentasaccharid-Einheit, die aus drei Mannose- und zwei N-Acetylglucosaminresten besteht. Zur weiteren Modifikation der Kohlenhydrat-Einheiten werden die Proteine vom ER zum Golgi-Komplex transportiert. Die Struktur der N-gebundenen Oligosaccharideinheiten von Glykoproteinen wird von ihrer Konformation und der Zusammensetzung der Glykosyltransferasen der Golgi-Kompartimente bestimmt, in denen sie prozessiert werden.

Es wurde gezeigt, dass im Golgi-Komplex einiger Pflanzen-und Insektenzellen die Core-Pentasaccharideinheit durch Xylose und α1,3-gebundene Fucose substituiert wird (P. Lerouge et al. 1998 Plant Mol. Biol. 38, 31-48; Rayon et al. 1998 L. Exp. Bot. 49, 1463-1472). Das entstehende Heptasaccharid "MMXF³" stellt den Hauptoligosaccharid Typ in Pflanzen dar (Kurosaka et al. 1991 J.Biol.Chem., 266, 4168-4172). So weisen z.B. die Meerrettich Peroxidase, Karotten β-Fructosidase und Erythrina cristagalli Lectin als auch die Honigbienengift Phospholipase A2 oder die neuronalen Membran Glykoproteine aus Insekten Embryonen α1,3-Fucosereste, die an das Glykancore gebunden sind, auf. Diese Strukturen werden auch komplexe N-Glykane bzw. Mannose defiziente oder trunkierte N-Glykane genannt. Die α-Mannosylreste können weiters durch GlcNAc ersetzt werden, an die Galaktose und Fucose gebunden sind, so dass eine Struktur hergestellt wird, die dem humanen Lewis a Epitop entspricht (Melo et al. 1997, FEBS Lett. 415, 186-191; Fitchette-Laine et al. 1997 Plant J. 12, 1411-1417).

Weder die Xylose noch die α1,3-gebundene Fucose kommen in Säugerglykoproteinen vor. Es stellte sich heraus, dass die Core-α1,3-Fucose eine wichtige Rolle in der Epitoperkennung von Antikörpern spielt, die gegen pflanzliche und Insekten-N-gebundene Oligosaccharide gerichtet sind (I.B.H. Wilson et al., Glycobiology Vol. 8, Nr. 7, S. 651-661, 1998) und dadurch Immunreaktionen im menschlichen oder tierischen Körper gegen diese Oligosaccharide auslösen. Der α1,3-Fucoserest scheint weiters eine der Hauptursachen für die weitverbreitete allergische Kreuzreaktivität zwischen verschiedenen pflanzlichen und Insekten-Allergenen zu sein (Tretter et al., Int. Arch. Allergy Immunol. 1993; 102:259-266) und wird auch "kreuzreaktive Kohlenhydrat Determinante" (CCD) genannt. Bei einer Studie von Epitopen von Tomaten und Graspollen wurden ebenfalls α1,3-gebundene Fucoseresten als gemeinsame Determinante festgestellt, was der Grund für das häufige gemeinsame Auftreten von Tomaten- und Graspollenallergien in Patienten zu sein scheint (Petersen et al. 1996, J. Allergy Clin. Immunol., Vol 98, 4; 805-814). Die CCDs verschleiern weiters durch das häufige Auftreten von immunologischen Kreuzreaktionen Allergie-Diagnosen.

Diese immunologischen Reaktionen, die von pflanzlichen Proteinen im menschlichen Körper ausgelöst werden, sind das Hauptproblem bei der medizinischen Verwendung von in Pflanzen produzierten, rekombinanten menschlichen Proteinen. Um dieses Problem zu umgehen, müsste die α1,3 Core-Fucosylierung verhindert werden. Es konnte in einer Studien gezeigt werden, dass Oligosaccharide, die anstelle einer L-Fucose (6-Deoxy-L-Galaktose) eine L-Galaktose aufweisen, dennoch voll biologisch aktiv sind (E. Zablackis et al. 1996, Science, Vol 272). Gemäß einer anderen Studie wurde eine Mutante der Pflanze Arabidopsis thaliana isoliert, dem die N-Acetyl-Glucosaminyl-Transferase I fehlt, das erste Enzym in der Biosynthese von komplexen Glykanen. Die Biosynthese der komplexen Glykoproteine in dieser Mutante ist damit gestört. Dennoch sind diese mutierten Pflanzen in der Lage, sich unter bestimmten Bedingungen normal zu entwickeln (A. Schaewen et al. 1993, Plant Physiol. 102; 1109-1118).

Um die Bindung der Core-α1,3 Fucose in einem Oligosaccharid gezielt zu unterbinden, ohne auch in andere Glykosylierungsschritte einzugreifen, müsste nur das Enzym ausgeschalten werden, das direkt für diese spezifische Glykosylierung verantwortlich ist, nämlich die Core-α1,3-Fucosyltransferase. Sie wurde erstmals aus Mungo Bohnen isoliert und charakterisiert, wobei festgestellt wurde, dass die Aktivität dieses Enzyms abhängig von der Gegenwart von nichtreduzierenden GlcNAc-Enden ist (Staudacher et al., 1995, Glycoconjugate J. 12, 780-786). Diese Transferase, die nur in Pflanzen und Insekten, nicht aber im Menschen oder anderen Wirbeltieren vorkommt, müsste gezielt in aktiviert oder unterdrückt werden, so dass menschliche Proteine, die in Pflanzen oder pflanzlichen Zellen bzw. auch Insekten oder -zellen hergestellt werden, dieses Immunreaktionen auslösende Epitop nicht mehr wie bisher aufweisen.

Die Schrift von John M. Burke "Clearing the way for ribozymes" (Nature Biotechnology 15:414-415; 1997) betrifft die generelle Funktionsweise von Ribozymen.

Die Schrift von Pooga et al "Cell penetrating PNA constructs regulate galanin receptor levels and modify pain transmission in vivo" (Nature Biotechnology 16:857-861; 1998) betrifft PNA-Moleküle allgemein und spezifisch ein PNA-Molekül, das komplementär zur humanen Galaninrezeptortyp 1 mRNA ist.

Die US 5 272 066 A betrifft ein Verfahren zur Veränderung von eukaryotischen und prokaryotischen Proteinen, um ihre in vivo-Zirkulation zu verlängern. Dabei werden die gebundenen Oligosaccharide mit Hilfe von verschiedenen Enzymen, darunter auch die GlcNAc-α1→3 (4)-Fucosyltransferase verändert.

Die EP 0 643 132 A1 betrifft die Klonierung einer α1,3-Fucosyltransferase, die aus humanen Zellen (THP-1) isoliert wird. Die in dieser Schrift beschriebenen Kohlenwasserstoffketten entsprechen den menschlichen Sialyl Lewis x- und Sialyl Lewis a-Oligosacchariden. Die Spezifität des Enzyms aus humanen Zellen ist eine ganz andere als die Fucosyltransferase aus pflanzlichen Zellen.

Weitere tierische Fucosyltransferasen sind in Costache et al. (J. Biol. Chem. 272(47), (1997): 29721-29728) beschrieben.

Es ist ein Ziel der vorliegenden Erfindung, das Gen, das für eine pflanzliche Fucosyltransferase codiert, zu klonieren und zu sequenzieren, sowie Verfahren zur Verfügung zu stellen um Glykoproteine herzustellen, die die normalerweise vorkommende α1,3-Core Fucose nicht umfassen.

Die erfindungsgemäße Aufgabe wird gelöst durch ein Verfahren zur Herstellung rekombinanter Glykoproteine, umfassend die Produktion eines rekombinanten Glykoproteins in Pflanzen oder Pflanzenzellen, deren GlcNAc-α1,3-Fucosyltransferaseproduktion unterdrückt oder vollständig unterbunden ist und deren endogene GlcNAc-α1,3-Fucosyltransferase-Aktivität unter 50% der in natürlichen Pflanzen bzw. Pflanzenzellen vorkommenden GlcNAc-α1,3-Fucosyltransferase-Aktivität beträgt, und die GlcNAc-α-1,3-Fukosyltransferase durch ein DNA-Molekül, das eine Sequenz gemäß der SEQ ID No. 1 (in dieser Schrift wurde der IUPAC-Code verwendet, wobei "N" für Inosin steht) mit einem offenen Leserahmen von Basenpaar 211 bis Basenpaar 1740 umfasst oder zumindest 50 % Homologie zur oben genannten Sequenz aufweist oder unter stringenten Bedingungen mit der oben genannte Sequenz hybridisiert oder eine Sequenz umfasst, die in Folge des genetischen Codes zur oben genannte DNA-Sequenz degeneriert ist, codiert ist.

Diese Sequenz, die zuvor noch nie beschrieben wurde, eignet sich ausgezeichnet für jegliche Experimente, Analysen und Herstellungsverfahren etc., die sich auf die pflanzliche Fucosyl-transferase-Aktivität beziehen. Dabei ist sowohl die DNA-Sequenz als auch das von dieser Sequenz codierte Protein von Interesse, wobei aber insbesondere die DNA-Sequenz für die Unterbindung der Fucosyltransferase-Aktivität herangezogen wird.

Der offene Leserahmen der Sequenz mit der SEQ ID No: 1 codiert für ein Protein mit 510 Aminosäuren und mit einem theoretischen Molekulargewicht von 56,8 kDa, wobei im Bereich zwischen Asn36 und Gly54 vermutlich ein transmembranes Stück vorliegt. Der errechnete pI-Wert des codierten Proteins der Sequenz gemäß der SEQ ID No: 1 beträgt 7,51.

Die Aktivität der pflanzlichen Fucosyltransferase wird durch ein Verfahren nachgewiesen und gemessen, wobei die Fucosyltransferase zu einer Probe umfassend markierte Fucose und einen an einen Träger, z.B. Sepharose, gebundenen Akzeptor (z.B. ein Glykoprotein) zugesetzt wird. Nach der Reaktionszeit wird die Probe gewaschen und der Gehalt an gebundener Fucose gemessen. Die Aktivität der Fucosyltransferase wird dabei als positiv angesehen, wenn die Aktivitätsmessung zumindest um 10 bis 20%, insbesondere zumindest um 30 bis 50% höher liegt als die Aktivitätsmessung der Negativkontrolle. Die Struktur des Glykoproteins kann zusätzlich mittels HPLC verifiziert werden. Solche Protokolle sind Stand der Technik (Staudacher et al. 1998, Anal. Biochem. 246, 96-101; Staudacher et al. 1991, Eur.J. Biochem. 19.9, 745-751).

Beispielsweise wird Fucosyltransferase zu einer Probe umfassend radioaktiv markierter Fucose und einen Akzeptor, z.B. GlcNAcβ1-2Manα1-3(GlcNAcβ1-2Manα1-6)Manβ1-4GlcNAcβ1-4GlcNAcβ1-Asn, zugesetzt. Nach Reaktionszeit wird die Probe durch Anionenaustauschchromatographie gereinigt und der Gehalt an gebundener Fucose gemessen. Aus der Differenz der gemessenen Radioaktivität der Probe mit Akzeptor und der einer Negativkontrolle ohne Akzeptor kann die Aktivität errechnet werden. Die Aktivität der Fucosyltransferase wird bereits als positiv gewertet, wenn die gemessene Radioaktivität zumindest um 30-40% höher liegt als die gemessene Radioaktivität der Negativprobe.

Die Paarung von zwei DNA-Molekülen kann durch die Wahl der Temperatur und Ionenstärke der Probe verändert werden. Unter stringenten Bedingungen, werden erfindungegmäß Bedingungen verstanden, die eine exakte, stringente, Bindung ermöglichen. Z.B. werden die DNA-Moleküle in 7% Natrium Dodecyl Sulfat (SDS), 0,5M NaPO4 pH 7,0; 1mM EDTA bei 50°C hybridisiert und mit 1% SDS bei 42°C gewaschen.

Ob Sequenzen eine zumindest 50% Homologie zur SEQ ID No: 1 aufweisen, kann z.B. mit dem Programm FastDB der EMBL oder SWISSPROT Datenbank ermittelt werden.

Die Sequenz des DNA-Moleküls codiert für ein Protein mit einer GlcNAc-α1,3-Fucosyltransferase-Aktivität, insbesondere mit einer Core-α1,3-Fucosyltransferase-Aktivität. Wie oben bereits beschrieben, kommt die Core-α1,3-Fucosyltranferase in Pflanzen und Insekten, nicht aber im menschlichen Organismus vor, so dass insbesondere diese DNA-Sequenz geeignet ist, in Fucosyltransferase-spezifischen Analysen und Experimenten sowie Produktionsverfahren verwendet zu werden. Unter einer Core-α1,3-Fucosyltransferase wird insbesondere die GDP-L-Fuc:Asn-gebundene GlcNAc-α1,3-Fucosyltransferase verstanden. Im Rahmen der vorliegenden Erfindung ist mit der Bezeichnung α1,3-Fucosyl-transferase in der Regel insbesondere die Core-α1,3-Fucosyltransferase gemeint. Zur oben beschriebenen Aktivitätsmessung dienen dabei insbesondere Akzeptoren mit einem nicht reduzierenden GlcNAc-Ende. Solche Akzeptoren sind z.B. GlcNAcβ1- 2Manα1-3(GlcNAcβ1-2Manα1-6) Manβ1-4GlcNAcβ1-4GlcNAcβ1-Asn, GlcNAcβ1-2Manα1-3(GlcNAcβ1-2Manα1-6) Manβ1-4GlcNAcβ1-4 (Fucα1-6GlcNAcβ1-Asn und GlcNAcβ1-2Manα1-3[Manα1-3(Manα1-6) Manα1-6] Manβ1-4GlcNAcβ1-4GlcNAcβ1-Asn. Ob die Fucose gebunden ist, kann weiters durch Messen der Unempfindlichkeit gegenüber der N-Glykosidase F festgestellt werden, was mit Hilfe der Massenspektrometrie nachgewiesen werden kann.

Bevorzugterweise weist das DNA-Molekül zumindest 70-80%, besonders bevorzugt zumindest 95%, Homologie zur Sequenz gemäß der SEQ ID No: 1 auf. Diese Sequenz codiert für eine besonders aktive GlcNAc-α1,3-Fucosyltransferase. Da die DNA-Sequenz je nach Pflanze bzw. Insekt mehr oder weniger verändert sein kann, weist eine Sequenz, die z.B. 70% Homologie zur Sequenz gemäß der SEQ ID No: 1 aufweist, ebenfalls eine Fucosyltransferase-Aktivität auf, die ausreicht, um für Verfahren wie oben beschrieben verwendet zu werden.

Beschrieben wird ein DNA-Molekül das 2150 bis 2250, insbesondere 2198, Basenpaare umfasst. Dieses DNA-Molekül weist 100 bis 300, vorzugsweise 210, Basenpaare stromaufwärts vor dem Startcodon auf sowie 350 bis 440, insbesondere 458, Basenpaare stromabwärts nach dem Stopcodon des offenen Leserahmens auf, wobei das Ende des DNA-Moleküls vorzugsweise einen 3'-Poly(A)-Schwanz umfasst. Auf diese Weise ist eine einwandfreie Regulation auf dem Niveau der Translation gesichert, und es wird ein DNA-Molekül beschrieben, das besonders effizient und problemlos für eine aktive GlcNAc-α1,3-Fucosyltransferase codiert.

Die vorliegende Beschreibung offenbart weiters ein DNA-Molekül, das eine Sequenz gemäß der SEQ ID No: 3 umfasst oder eine Sequenz umfasst, die zumindest 85%, besonders bevorzugt zumindest 95%, insbesondere zumindest 99%, Homologie zur oben genannten Sequenz aufweist oder unter stringenten Bedingungen mit der oben genannten Sequenz hybridisiert oder die infolge des genetischen Codes zur oben genannten DNA Sequenz degeneriert ist. Die Homologie wird vorzugsweise mit einem Programm bestimmt, das Insertionen und Deletionen erkennt und diese nicht in der Homologie-Berechnung berücksichtigt. Diese Nukleotidsequenz codiert für ein konserviertes Peptid-Motiv, d.h. dass die Mehrheit der aktiven und funktionierenden GlcNAc-α1,3-Fucosyltransferasen die dadurch codierte Aminosäurensequenz umfasst. Die Sequenz kann dabei sowohl die gleiche Größe wie die Sequenz gemäß der SEQ ID No: 3 aufweisen, oder aber selbstverständlich auch größer sein. Diese Sequenz weist eine geringere Länge auf als die Sequenz, die für das gesamte Protein codiert, und ist somit weniger an fällig für Rekombinationen, Deletionen oder andere Mutationen. Aufgrund des konservierten Motivs und ihrer erhöhten Stabilität eignet sich diese Sequenz besonders gut für Sequenzerkennungs--Tests.

Die SEQ ID No: 3 weist folgende Sequenz auf: 5'-gaagccctgaagcactacaaatttagcttagcgtttgaaaattcgaatgaggaag attatgtaactgaaaaattcttccaatcccttgttgctggaactgtccct -3'

Offenbart wird ein DNA-Molekül, das eine Teilsequenz eines der oben genannten DNA-Moleküle umfasst und eine Größe von 20 bis 200, vorzugsweise 30 bis 50, Basenpaaren aufweist. Das DNA-Molekül kann beispielsweise dazu eingesetzt werden, als Sonde an komplementäre Sequenzen von GlcNAc-α1,3-Fucosyltransferasen zu binden, so dass diese aus einer Probe selektiert werden können. Auf diese Weise können weitere GlcNAc-α1,3-Fucosyltransferasen aus den verschiedensten Pflanzen und Insekten selektiert, isoliert und charakterisiert werden. Es können jede beliebige oder auch mehrere verschiedene Teilsequenzen verwendet werden, insbesondere ein Teil des oben schon beschriebenen konservierten Motivs.

Dabei ist es besonders vorteilhaft, wenn eines der oben genannten DNA-Moleküle kovalent mit einer nachweisbaren Markierungssubstanz assoziiert ist. Als Markierungssubstanz kommt jeder gebräuchliche Marker in Frage, so z.B. fluoreszierende, luminiszierende, radioaktive Marker, nicht-isotope Markierungen, wie Biotin, etc. Dadurch werden Reagenzien bereitgestellt, die für den Nachweis, die Selektion und Quantifizierung von entsprechenden DNA-Molekülen in festen Gewebeproben (z.B. von Pflanzen) oder auch in Flüssigkeitsproben durch Hybridisierungsverfahren nützlich sind.

Weiters offenbart wird ein biologisch funktioneller Vektor, der eines der oben genannten DNA-Moleküle oder Teile unterschiedlicher Länge davon, mit mindestens 20 Basenpaaren umfasst. Zur Transfektion in Wirtszellen ist ein eigenständiger, vermehrungsfähiger Vektor notwendig, wobei je nach Wirtszelle, Transfektionsmechanismus, Aufgabe und Größe des DNA-Moleküls ein passender Vektor verwendet werden kann. Da eine große Zahl an verschiedenen Vektoren bekannt ist, würde eine Aufzählung den Rahmen der Anmeldung sprengen, so dass hier darauf verzichtet wird, insbesondere, da die Vektoren dem Fachmann bestens bekannt sind (bezüglich Vektoren sowie allen in dieser Schrift verwendeten Techniken und Begriffe, die dem Fachmann bekannt sind, siehe auch Sambrook Maniatis). Idealerweise weist der Vektor eine geringe Molekülmasse auf und sollte selektierbare Gene aufweisen, um in einer Zelle zu einem leicht erkennbaren Phänotyp zu führen, so dass eine einfache Selektion von vektorhältigen und vektorfreien Wirtszellen möglich ist. Um eine hohe Ausbeute an DNA und entsprechenden Genprodukten zu erhalten, sollte der Vektor einen starken Promotor, sowie einen Enhancer, Genamplifikationssignale und Regulatorsequenzen aufweisen. Für eine autonome Replikation des Vektors ist weiters ein Replikationsursprung wichtig. Polyadenylierungsstellen sind für eine korrekte Prozessierung der mRNA und Spleißsignale für die von RNA-Transcripten verantwortlich. Werden Phagen, Viren oder Viruspartikel als Vektoren verwendet, steuern Verpackungssignale die Verpackung der Vektor-DNA. Z.B. sind für die Transkription in Pflanzen Ti-Plasmide geeignet und für die Transkription in Insektenzellen Baculoviren, bzw. in Insekten Transposons, wie das P Element.

Wird der oben beschriebene Vektor in eine Pflanze oder Pflanzenzelle eingeschleust, so wird eine posttranskriptionelle Unterdrückung der Genexpression des endogenen α1,3-Fucosyltransferase-Gens durch Transkription eines zu diesem homologen Transgens oder Teilen davon in Sense-Orientierung erreicht. Für diese Sense-Technik wird weiters auf die Schriften Baulcombe 1996, Plant.Mol.Biol. 9:373-382 und Brigneti et al. 1998, EMBO J. 17:6739-6746 verwiesen. Diese Strategie des "Gen- Silencing" stellt eine wirkungsvolle Möglichkeit dar, die Expression des α1,3-Fucosyltransferase-Gens zu unterdrücken, s. auch Waterhouse et al. 1998, Proc.Natl.Acad.Sci.USA, 95:13959- 13964.

Weiters betrifft die Offenbarung einen biologisch funktionellen Vektor, der ein DNA-Molekül gemäß einem der oben beschriebenen Ausführungsformen oder Teile unterschiedlicher Länge davon in inverser Orientierung zum Promotor umfasst. Wird dieser Vektor in eine Wirtszelle transfektiert, wird eine "Antisense-mRNA" abgelesen, die komplementär zur mRNA der GlcNAc α1,3-Fucosyltransferase ist und diese komplexiert. Diese Bindung behindert entweder die korrekte Prozessierung, den Transport, die Stabilität, oder durch Verhinderung der Ribosomenanlagerung die Translation und damit die normale Genexpression der GlcNAc α1,3-Fucosyltransferase.

Obwohl die gesamte Sequenz des DNA-Moleküls in den Vektor eingebaut werden könnte, können Teilsequenzen davon aufgrund der geringeren Größe für bestimmte Zwecke vorteilhafter sein. Wichtig ist z.B. beim Antisense-Aspekt, dass das DNA-Molekül groß genug ist, um eine ausreichend große Antisense-mRNA zu bilden, die an die Transferase-mRNA bindet. Beispielsweise umfasst ein geeignetes Antisense-RNA-Molekül 50 bis 200 Nukleotide, da viele der bekannten natürlich vorkommenden Antisense-RNA-Molekülen etwa 100 Nukleotide umfassen.

Für eine besonders effektive Inhibierung der Expression einer aktiven α1,3-Fucosyltransferase ist eine Kombination der Sense-Technik und Antisense-Technik geeignet (Waterhouse et al. 1998, Proc.Natl.Acad.Sci.USA, 95:13959-13964).

Vorteilhafterweise werden schnell hybridisierende RNA-Moleküle eingesetzt. Die Effizienz von Antisense-RNA-Molekülen, die eine Größe von über 50 Nukleotiden aufweisen, hängt von der Anlagerungskinetik in vitro ab. So zeigen z.B. schnell anlagernde Antisense-RNA-Moleküle eine stärkere Inhibition der Proteinexprimierung als langsam hybridisierende RNA-Moleküle (Wagner et al. 1994, Annu. Rev. Microbiol., 48:713-742; Rittner et al. 1993, Nucl. Acids Res, 21:1381-1387). Solche schnell hybridisierenden Antisense-RNA-Moleküle weisen insbesondere eine große Anzahl von externen Basen (freie Enden und Verbindungssequenzen), eine große Anzahl von strukturellen Subdomänen (Komponenten), sowie einen geringen Grad an Schleifen (Patzel et al. 1998; Nature Biotechnology, 16; 64-68) auf. Die hypothetischen Sekundärstrukturen des Antisense-RNA-Moleküls können z.B. mit Hilfe eines Computerprogramms ermittelt werden, gemäß dem eine geeignete Antisense-RNA DNA-Sequenz ausgesucht wird.

Es können unterschiedliche Sequenzregionen des DNA-Moleküls in den Vektor eingebaut werden. Eine Möglichkeit besteht z.B. darin, nur den Teil, der für die Ribosomenanlagerung verantwortlich ist, in den Vektor einzubauen. Eine Blockierung in diesem Bereich der mRNA reicht aus, um die gesamte Translation zu stoppen. Eine besonders hohe Effizienz der Antisense-Moleküle ergibt sich auch für die 5'- und 3'-untranslatierten Regionen des Gens.

Ein genanntes DNA-Molekül kann eine Sequenz aufweisen, die eine Deletions-, Insertions- und/oder Substitutionsmutation umfasst. Die Anzahl der mutierten Nukleotide ist dabei variabel und reicht von einem einzigen bis zu mehreren deletierten, inserierten oder substituierten Nukleotiden. Es ist auch möglich, dass durch die Mutation der Leserahmen verschoben ist. Wichtig bei einem derartigen "Knockout-Gen" ist lediglich, dass die Expression einer GlcNAc-α1,3-Fucosyltransferase gestört ist und die Bildung eines aktiven, funktionellen Enzyms verhindert wird. Dabei ist die Stelle der Mutation variabel, solange die Exprimierung eines enzymatisch aktiven Proteins unterbunden ist. Vorzugsweise ist die Mutation im katalytischen Bereich des Enzyms, der sich im C-terminalen Bereich befindet. Die Verfahren zum Einführen von Mutationen in DNA-Sequenzen sind dem Fachmann bestens bekannt, so dass hier darauf verzichtet wird, näher auf die verschiedenen Möglichkeiten der Mutagenesen einzugehen. Es können sowohl zufällige Mutagenesen aber auch insbesondere zielgerichtete Mutagenesen, z.B. die site-directed-mutagenesis, die oligonukleotidgesteuerte Mutagenese oder Mutagenesen mit Hilfe von Restriktionsenzymen hier zur Anwendung kommen.

Weiters beschrieben ist ein DNA-Molekül, das für ein Ribozym codiert, welches zwei Sequenzabschnitte von jeweils mindestens 10 bis 15 Basenpaaren aufweist, die Sequenzabschnitten eines DNA-Moleküls wie oben beschrieben komplementär sind, so dass das Ribozym die mRNA komplexiert und schneidet, die von einem natürlichen GlcNAc-α1,3 Fucosyltransferase DNA-Molekül transkribiert wird. Das Ribozym erkennt die mRNA der GlcNAc-α1,3 Fucosyltransferase durch komplementäre Basenpaarung mit der mRNA. Anschließend schneidet und zerstört das Ribozym die RNA in einer sequenzspezifischen Art, bevor das Enzym translatiert wird. Nach Dissoziation vom geschnittenen Substrat, hybridisiert das Ribozym wiederholt mit RNA-Molekülen und wirkt als spezifische Endonuklease. Generell können Ribozyme spezifisch zur Inaktivierung einer bestimmten mRNA hergestellt werden, selbst wenn nicht die gesamte DNA-Sequenz, die für das Protein codiert, bekannt ist. Ribozyme sind besonders dann effizient, wenn die Ribosomen langsam an der mRNA entlang gleiten. In diesem Fall ist es für das Ribozym leichter, eine Ribosomen-freie Stelle an der mRNA zu finden. Aus diesem Grund sind langsame Ribosomen-Mutante als System für Ribozyme ebenfalls geeignet (J.Burke, 1997, Nature Biotechnology; 15, 414-415). Dieses DNA-Molekül eignet sich besonders gut für die Unterdrückung bzw. Unterbindung der Exprimierung einer pflanzlichen GlcNAc-α1,3-Fucosyltransferase.

Eine Möglichkeit besteht auch darin, eine abgewandelte Form eines Ribozyms einzusetzen, nämlich ein Minizym. Insbesondere für das Schneiden von größeren mRNA-Molekülen sind Minizyme effizient. Ein Minizym ist ein Hammerkopf-Ribozym, das anstelle der Stem/Loop II einen kurzen Oligonukleotid-Linker aufweist. Besonders effizient sind Dimer-Minizyme (Kuwabara et al. 1998, Nature Biotechnology, 16; 961-965).

Demnach wird ein biologisch funktioneller Vektor beschrieben, der eines der beiden zuletztgenannten DNA- Moleküle (Mutations oder Ribozym-DNA-Molekül) umfasst. Hierbei gilt das oben bezüglich Vektoren bereits Beschriebene. Ein solcher Vektor kann z.B. in einen Mikroorganismus eingeschleust und für die Herstellung von hohen Konzentrationen der oben beschriebenen DNA-Moleküle verwendet werden. Weiters ist ein solcher Vektor besonders gut für die Einschleusung des spezifischen DNA-Moleküls in einen pflanzlichen oder Insekten-Organismus geeignet, um die GlcNAc-α1,3-Fucosyltransferase-Produktion in diesem Organismus einzuschränken oder völlig zu unterbinden.

Offenbart wird en Verfahren zur Herstellung einer cDNA umfassend das offenbarte DNA-Molekül, wobei RNA aus Insekten- bzw. pflanzlichen Zellen, insbesondere aus Hypokotyl-Zellen, isoliert wird, mit der nach Zusetzen einer reversen Transkriptase und Primern eine reverse Transkription durchgeführt wird. Die einzelnen Schritte dieses Verfahrens werden nach Protokollen wie an sich bekannt durchgeführt. Für die reverse Transkription besteht einerseits die Möglichkeit, mit Hilfe von Oligo(dT)-Primern die cDNA der gesamten mRNA herzustellen und erst anschließend mit ausgewählten Primern eine PCR durchzuführen, um DNA-Moleküle umfassend das GlcNAc-α1,3-Fucosyltransferase-Gen herzustellen. Andererseits können die ausgewählten Primer direkt für die reverse Transkription eingesetzt werden, um eine kurze, spezifische cDNA zu erhalten. Die geeigneten Primer können z.B. synthetisch nach Vorlage von cDNA-Sequenzen der Transferase hergestellt werden. Mit Hilfe dieses Verfahrens können sehr rasch und einfach, mit geringer Fehlerquote, große Mengen an cDNA-Molekülen hergestellt werden.

Weiters offenbart wird ein Verfahren zum Klonieren einer GlcNAc-α1,3-Fucosyltransferase, dadurch gekennzeichnet, dass das DNA-Molekül in einen Vektor kloniert wird, der anschließend in eine Wirtszelle bzw. einen Wirt transfektiert wird, wobei durch Selektion und Amplifikation von transfektierten Wirtszellen Zellinien erhalten werden, die die aktive GlcNAc-α1,3-Fucosyltransferase exprimieren. Das DNA-Molekül wird beispielsweise mit Hilfe von Restriktionsendonukleasen in den Vektor eingefügt. Für den Vektor gilt wiederum das oben bereits angeführte. Wichtig ist bei diesem Verfahren, dass ein effizientes Wirt-Vektor System gewählt wird. Um ein aktives Enzym zu erhalten, sind eukaryotische Wirtszellen besonders geeignet. Eine Möglichkeit besteht darin, den Vektor in Insekten-Zellen zu transfektieren. Dabei wäre insbesondere ein Insektenvirus als Vektor zu verwenden, so z.B. Baculovirus.

Selbstverständlich können menschliche oder andere Wirbeltier-Zellen ebenfalls transfektiert werden, wobei diese ein ihnen fremdes Enzym exprimieren würden.

Offenbart wird ein Verfahren zur Herstellung von rekombinanten Wirtszellen, insbesondere Pflanzen- oder Insektenzellen, bzw. Pflanzen oder Insekten mit einer unterdrückten bzw. vollständig unterbundenen GlcNAc-α1,3-Fucosyltransferase-Produktion, das dadurch gekennzeichnet ist, dass zumindest einer der Vektoren, nämlich der umfassend das erfindungsgemäße DNA-Molekül, das mutierte DNA-Molekül oder das DNA-Molekül, das für Ribozyme codiert oder der umfassend das DNA-Molekül in inverser Orientierung zum Promotor, in die Wirtszelle bzw. Pflanze oder in das Insekt eingeschleust wird. Hier gilt ebenfalls das oben für die Transfektion bereits Beschriebene.

Als Wirtszellen können z.B. Pflanzenzellen verwendet werden, wobei hier beispielsweise das Ti-Plasmid mit dem Agrobacterium-System in Frage kommt. Es ist mit dem Agrobakterium-System möglich, direkt eine Pflanze zu transfektieren: Agrobakterien verursachen bei Pflanzen Wurzelhalsgallen. Wenn Agrobakterien eine verletzte Pflanze infizieren, gelangen die Bakterien selbst nicht in die Pflanze, sondern sie schleusen den rekombinanten DNA-Abschnitt, die sogenannte T-DNA aus dem ringförmigen, extrachromosomalen, tumorinduzierenden Ti-Plasmid in die Pflanzenzellen ein. Die T-DNA, und damit auch das darin eingefügte DNA-Molekül, wird stabil in die chromosomale DNA der Zelle eingebaut, so dass die Gene der T-DNA in der Pflanze exprimiert werden.

Es gibt zahlreiche bekannte, effiziente Transfektionsmechanismen für verschiedene Wirtssysteme. Einige Beispiele sind Elektroporation, die Calciumphosphatmethode, Mikroinjektion, Liposomenmethode.

Die transfektierten Zellen werden anschließend selektiert, z.B. aufgrund von Antibiotika-Resistenzen, für die der Vektor Gene aufweist, oder anderer Markergene. Danach werden die transfektierten Zellinien amplifiziert, entweder in kleineren Mengen, z.B. in Petrischalen, oder in großen Mengen, etwa in Fermentoren. Weiters weisen Pflanzen eine besondere Eigenschaft auf, sie sind nämlich in der Lage sich aus einer (transfektierten) Zelle bzw. aus einem Protoplasten zu einer vollständigen Pflanze wieder zu entwickeln, die gezüchtet werden kann.

Je nach eingesetztem Vektor kommt es zu Vorgängen im Wirt, so dass die Enzym-Expression unterdrückt oder vollständig unterbunden wird:
Wird der Vektor umfassend das DNA-Molekül mit der Deletions- Insertions- oder Substitutionsmutation transfektiert, so kommt es zu einer homologen Rekombination : Das mutierte DNA-Mo-lekül erkennt trotz Mutation die identische Sequenz im Genom der Wirtszelle und wird an genau der Stelle eingebaut, so dass ein "Knockout-Gen" entsteht. Auf diese Weise wird in das Gen für die GlcNAc-α1,3-Fucosyltransferase eine Mutation eingebracht, die die einwandfreie Expression der GlcNAc-α1,3-Fucosyltransferase inhibieren kann. Wie oben schon dargelegt ist es bei dieser Technik wichtig, dass die Mutation ausreicht, um die Expression des aktiven Proteins zu unterbinden. Nach Selektion und Amplifikation kann als zusätzliche Überprüfung das Gen sequenziert werden, um den Erfolg der homologen Rekombination bzw. den Grad der Mutation festzustellen.
Wird der Vektor umfassend das DNA-Molekül, das für ein Ribozym codiert, transfektiert, so wird in der Wirtszelle das aktive Ribozym exprimiert. Das Ribozym komplexiert die komplementäre mRNA Sequenz der GlcNAc-α1,3-Fucosyltransferase zumindest an einer bestimmten Stelle, schneidet diese Stelle und kann auf diese Weise die Translation des Enzyms inhibieren. In dieser Wirtszelle sowie in den von ihr abstammenden Zellinien bzw. gegebenenfalls Pflanze wird keine GlcNAc-α1,3-Fucosyltransferase exprimiert.

Im Falle des Vektors umfassend das offenbarte DNA-Molekül in Sense- oder inverser Richtung zum Promotor, wird in der transfektierten Zelle (bzw. Pflanze) eine Sense- oder Antisense-mRNA exprimiert. Die Antisense-mRNA ist komplementär zu zumindest einem Teil der mRNA-Sequenz der GlcNAc-α1,3-Fucosyltransferase und kann ebenfalls die Translation des Enzyms inhibieren. Als Beispiel für ein Verfahren zur Unterdrückung der Expression eines Gens durch Antisense-Technik wird auf die Schrift Smith et al. 1990, Mol.Gen.Genet. 224:477-481 verwiesen, wobei in dieser Schrift die Expression eines Gens, das im Reifungsprozess bei Tomaten involviert ist, inhibiert wird.

In allen Systemen wird die Expression der GlcNAc-α1,3-Fucosyltransferase zumindest unterdrückt, vorzugsweise sogar vollständig unterbunden. Der Grad der Störung der Genexpression hängt vom Grad der Komplexierung, homologen Rekombination, von eventuellen anschließenden zufälligen Mutationen, und sonstigen Vorgängen im Bereich des Genoms ab. Die transfektierten Zellen werden auf GlcNAc-α1,3-Fucosyltransferase Aktivität überprüft und selektiert.

Es besteht weiters die Möglichkeit, die oben beschriebene Unterdrückung der Expression der α1,3-Fucosyltransferase noch weiter zu verstärken, indem zusätzlich zur Einschleusung eines oben beschriebenen Vektors, ein Vektor umfassend ein Gen, das für ein Säugetier-Protein, z.B. β1,4-Galaktosyltransferase, codiert, in den Wirt eingeschleust wird. Die Fukosylierung kann durch die Wirkung anderer Säugetier-Enzyme vermindert werden, wobei die Kombination der Inhibierung der Exprimierung einer aktiven α1,3-Fucosyltransferase mit Hilfe eines erfindungsgemäßen Vektors und mit Hilfe eines Säugetier-Enzym-Vektors besonders effizient ist.

Für die Transfektion kann jegliche Pflanzensorte eingesetzt werden, beispielsweise die Mungo Bohne, Tabakpflanze, Tomaten-und/oder Kartoffelpflanze.

Ein anderes, vorteilhaftes Verfahren zur Herstellung von rekombinanten Wirtszellen, insbesondere Pflanzen- oder Insektenzellen bzw. Pflanzen oder Insekten, besteht darin, dass das DNA-Molekül umfassend die Mutation in das Genom der Wirtszelle bzw. Pflanze oder des Insekts an der Stelle der nichtmutierten, homologen Sequenz eingeschleust wird (Schaefer et al. 1997, Plant J.; 11 (6): 1195-1206). Dieses Verfahren funktioniert demnach nicht mit einem Vektor, sondern mit einem reinen DNA-Molekül. Das DNA-Molekül wird z.B. durch Genbombardement, Mikroinjektion oder Elektroporation, um nur drei Beispiele zu nennen, in den Wirt eingeschleust. Wie oben bereits dargelegt, legt sich das DNA-Molekül an die homologe Sequenz im Genom des Wirten an, so dass es zu einer homologen Rekombination und damit zur Aufnahme der Deletions-, Insertions- bzw. Substitutionsmutation im Genom kommt: Die Exprimierung der GlcNAc-α1,3-Fucosyltransferase kann unterdrückt bzw. vollständig unterbunden werden.

Beschrieben sind Pflanzen bzw. Pflanzenzellen sowie Insekten bzw. Insektenzellen, wobei ihre GlcNAc-α1,3-Fucosyltransferase-Aktivität unter 50%, insbesondere unter 20%, besonders bevorzugt 0%, der in natürlichen Pflanzen bzw. Pflanzenzellen und Insekten bzw. Insektenzellen vorkommende GlcNAc-α1,3-Fucosyltransferase-Aktivität beträgt. Der Vorteil dieser Pflanzen bzw. Pflanzenzellen liegt darin, dass die Glykoproteine, die von ihnen produziert werden, keine bzw. kaum α1,3-gebundene Fucose aufweisen. Werden nun Produkte dieser Pflanzen bzw. Insekten vom menschlichen oder Wirbeltier-Körper aufgenommen, kommt es zu keiner Immunreaktion gegen das α1,3-Fucose-Epitop.

Beschrieben werden rekombinante Pflanzen bzw. Pflanzenzellen, die nach einem der oben beschriebenen Verfahren hergestellt sind und dass ihre GlcNAc-α1,3-Fucosyltransferaseproduktion unterdrückt bzw. vollständig unterbunden ist.

Offenbart werden ebenfalls rekombinante Insekten bzw. Insektenzellen, die nach einem der oben beschriebenen Verfahren hergestellt sind und dass ihre GlcNAc α1,3-Fucosyltransferaseproduktion unterdrückt bzw. vollständig unterbunden ist. Auch hier werden keine Glykoproteine mit α1,3-gebundenen Fucoseresten produziert, so dass es ebenfalls zu keiner Immunreaktion gegen das α1,3-Fucose-Epitop kommt.

Genannt wird auch ein PNA-Molekül, das eine Basensequenz umfasst, die komplementär zur Sequenz des DNA-Moleküls sowie Teilsequenzen davon ist. PNA (Peptid Nukleinsäure) ist eine DNA ähnliche Sequenz, wobei die Nukleobasen an ein Pseudopeptidrückgrat gebunden sind. PNA hybridisiert im allgemeinen mit komplementären DNA-, RNA- oder PNA-Oligomeren durch Watson-Crick Basenpaarung und Helixformation. Das Peptidrückgrat gewährleistet eine größere Resistenz gegenüber enzymatischer Degradation. Das PNA-Molekül stellt dadurch ein verbessertes Antisense-Agens dar. Weder Nukleasen noch Proteasen sind in der Lage, ein PNA-Molekül anzugreifen. Die Stabilität des PNA-Moleküls, wenn es an eine komplementäre Sequenz gebunden ist, weist eine ausreichende sterische Blockierung von DNA und RNA Polymerasen, der reversen Transkriptase, Telomerase und der Ribosome.

Weist das PNA-Molekül die oben genannte Sequenz auf, so bindet sie an die DNA bzw. an ein Stelle der DNA, die für GlcNAc-α1,3-Fucosyltransferase codiert, und kann auf diese Weise die Transkription dieses Enzyms inhibieren. Das PNA-Molekül, da es weder transkribiert noch translatiert wird, wird synthetisch hergestellt, z.B. mit Hilfe der t-Boc-Technik.

Offenbart wird ein PNA-Molekül, das eine Basensequenz umfasst, die der Sequenz des DNA-Moleküls sowie Teilsequenzen davon entspricht. Dieses PNA-Molekül komplexiert die mRNA bzw. eine Stelle der mRNA der GlcNAc-α1,3-Fucosyltransferase, so dass die Translation des Enzyms inhibiert ist. Hier trifft ähnliches, wie für die Antisense-RNA zu. So ist z.B. ein besonders effizienter Komplexierungsbereich die Translationsstart-Region oder auch die 5'-nichttranslatierten Regionen der mRNA.

Offenbart wird ein Verfahren zur Herstellung von Pflanzen oder Insekten bzw. Zellen, insbesondere Pflanzen- oder Insektenzellen, die eine blockierte Expression der GlcNAc-α1,3-Fucosyltransferase auf dem Niveau der Transkription bzw. Translation aufweisen, das dadurch gekennzeichnet ist, dass PNA-Moleküle in die Zellen eingeschleust werden. Um das PNA-Molekül bzw. die PNA-Moleküle in die Zelle einzuschleusen, werden wiederum die herkömmlichen Methoden, wie z.B. Elektroporation oder Mikroinjektion angewandt. Besonders effizient ist das Einschleusen, wenn die PNA-Oligomere an Zellen-Penetrationspeptide, z.B. Transportan oder pAntp, gebunden sind (Pooga et al. 1998, Nature Biotechnology, 16; 857-861).

Die Erfindung stellt ein Verfahren zur Herstellung von rekombinanten Glykoproteinen zur Verfügung, das dadurch gekennzeichnet ist, dass die rekombinanten Pflanzen bzw. Pflanzenzellen, deren GlcNAc-α1,3-Fucosyltransferase-Produktion unterdrückt bzw. vollständig unterbunden ist, mit dem Gen, das für das Glykoprotein codiert, transfektiert sind, so dass die rekombinanten Glykoproteine exprimiert werden. Dabei werden wie oben schon beschriebene Vektoren umfassend Gene für die gewünschten Proteine in den Wirt bzw. Wirtszellen wie ebenfalls oben schon beschrieben transfektiert. Die transfektierten Pflanzen- oder Insektenzellen exprimieren die gewünschten Proteine, wobei sie keine oder kaum α1,3-gebundene Fucose aufweisen. Dadurch lösen sie die oben bereits erwähnten Immunraktionen im menschlichen oder Wirbeltier-Körper nicht aus. Es können jegliche Proteine in diesen Systemen produziert werden.

Vorzugsweise wird ein Verfahren zur Herstellung von rekombinanten humanen Glykoproteinen zur Verfügung gestellt, das dadurch gekennzeichnet ist, dass die rekombinanten Pflanzen bzw. Pflanzenzellen, deren GlcNAc-α1,3-Fucosyltransferaseproduktion unterdrückt bzw. vollständig unterbunden ist, mit dem Gen, das für das Glykoprotein codiert, transfektiert sind, so dass die rekombinanten Glykoproteine exprimiert werden. Durch dieses Verfahren wird es möglich, menschliche Proteine in Pflanzen(zellen) zu produzieren, die, wenn sie durch den menschlichen Körper aufgenommen sind, keine gegen α1,3-gebundene Fucosereste gerichtete Immunreaktion auslösen. Dabei besteht die Möglichkeit, Pflanzensorten zur Produktion der rekombinanten Glykoproteine einzusetzen, die als Nahrungsmittel dienen, z.B. Banane, Kartoffel und/oder Tomate. Die Gewebe dieser Pflanze umfassen das rekombinante Glykoprotein, so dass z.B. durch Extraktion des rekombinanten Glykoproteins aus dem Gewebe und anschließende Verabreichung bzw. direkt durch den Verzehr des pflanzlichen Gewebes das rekombinante Glykoprotein in den menschlichen Körper aufgenommen wird.

Bevorzugterweise ist dabei ein Verfahren zur Herstellung von rekombinanten humanen Glykoproteinen zur medizinischen Verwendung, das die erfindungsgemäßen, rekombinanten Pflanzen bzw. Pflanzenzellen, deren GlcNAc-α1,3-Fucosyltransferase-Produktion unterdrückt bzw. vollständig unterbunden ist, mit dem Gen, das für das Glykoprotein codiert, transfektiert sind, so dass die rekombinanten Glykoproteine exprimiert werden. Hierbei kommt jegliches Protein in Frage, das medizinisch von Interesse ist.

Weiters betrifft die vorliegende Erfindung rekombinante Glykoproteine, wobei sie gemäß einem oben beschriebenen Verfahren in pflanzlichen oder Insekten-Systemen hergestellt wurden und wobei ihre Peptidsequenz unter 50%, insbesondere unter 20%, besonders bevorzugt 0%, der in den nicht Fucosyltransferase reduzierten pflanzlichen oder Insekten-Systemen exprimierten Proteinen vorkommenden α1,3-gebundenen Fucose-Reste aufweist. Vorzuziehen sind natürlich Glykoproteine, die keine α1,3-gebundenen Fucose-Reste aufweisen. Die Menge an α1,3-gebundener Fucose ist abhängig von dem Grad der oben beschriebenen Unterdrückung der GlcNAc-α1,3-Fucosyltransferase.

Bevorzugterweise betrifft die Erfindung rekombinante humane Glykoproteine, die gemäß einem oben beschriebenen Verfahren in pflanzlichen Systemen hergestellt wurden, und die in ihrer Peptidsequenz unter 50%, insbesondere unter 20%, besonders bevorzugt 0%, der in den nicht Fucosyltransferase reduzierten pflanzlichen Systemen exprimierten Proteinen vorkommenden α1,3-gebundenen Fucose-Reste aufweisen.

Beschrieben werden rekombinante humane Glykoproteine zur medizinischen Verwendung, die gemäß einem oben beschriebenen Verfahren in pflanzlichen oder Insekten-Systemen hergestellt wurden, und die in ihrer Peptidsequenz unter 50%, insbesondere unter 20%, besonders bevorzugt 0%, der in den nicht Fucosyltransferase reduzierten pflanzlichen oder Insekten-Systemen exprimierten Proteinen vorkommenden α1,3-gebundenen Fucose-Reste aufweisen.

Diese Glykoproteine können andere gebundene Oligosaccharid-Einheiten, die für Pflanzen bzw. Insekten spezifisch sind, aufweisen, wodurch sie sich - im Falle von humanen Glykoproteinen - von diesen natürlichen Glykoproteinen unter scheiden. Nichtsdestotrotz werden durch die Glykoproteine eine geringere bzw. gar keine Immunreaktion im menschlichen Körper ausgelöst, da, wie in der Beschreibungseinleitung schon dargestellt, die α1,3-gebundenen Fucose-Reste die Hauptursache der Immunreaktionen bzw. Kreuz-Immunreaktionen gegen pflanzliche und Insekten-Glykoproteine sind.

Offenbart wird eine pharmazeutische Zusammensetzung, die die Glykoproteine umfasst. Zusätzlich zu den Glykoproteinen umfasst die pharmazeutische Zusammensetzung weitere, für solche Zusammensetzungen übliche Zusätze. Dies sind z.B. geeignete Verdünnungsmittel verschiedenen Puffergehalts (z.B. Tris-HCl, Acetat, Phosphat), pH und Ionenstärke, Additive, wie etwa Tenside und Löslichmacher (z.B. Tween 80, Polysorbate 80), Konservierungsmittel (z.B. Thimerosal, Benzylalkohol), Adjuvantien, Antioxidationsmittel (z.B. Ascorbinsäure, Natriummetabisulfit), Emulgatoren, Füllstoffe (z.B. Lactose, Mannitol), kovalente Bindung von Polymeren, wie etwa Polyethylenglykol, an das Protein, Einbau des Materials in teilchenförmige Zubereitungen von polymeren Verbindungen, wie etwa Polymilchsäure, Polyglykolsäure, etc. oder in Liposome, Hilfsstoffe und/oder Trägerstoffe, die bei der jeweiligen Behandlung nützlich sind. Solche Zusammensetzungen werden den physikalischen Zustand, die Stabilität, die Geschwindigkeit der in-vivo Freisetzung und die Geschwindigkeit der in-vivo Ausscheidung der Glykoproteine beeinflussen.

Beschrieben wird auch ein Verfahren zum Selektieren von DNA-Molekülen, die für eine GlcNAc-α1,3-Fucosyltransferase co-dieren, in einer Probe, wobei die offenbarten, markierten DNA-Moleküle zur Probe zugesetzt werden, die an die DNA-Moleküle, die für eine GlcNAc-α1,3-Fucosyltransferase codieren, binden. Die hybridisierten DNA-Moleküle können detektiert, quantifiziert und selektiert werden. Damit die Probe Einzelstrang-DNA aufweist, mit der die markierten DNA-Moleküle hybridisieren können, wird die Probe, z.B. durch Erwärmen, denaturiert.

Eine Möglichkeit besteht darin, die zu untersuchende DNA, eventuell nach Zusetzen von Endonukleasen, durch Gelelektrophorese auf einem Agarosegel zu trennen. Nach Überführen auf eine Membran aus Nitrozellulose werden die markierten DNA-Moleküle zugesetzt, die an das entsprechende homologe DNA-Molekül hybridisieren ("Southern Blotting").

Eine andere Möglichkeit besteht darin, homologe Gene aus anderen Spezien durch PCR-abhängige Verfahren unter Verwendung von spezifischen und/oder degenerierten Primern, abgeleitet aus der Sequenz des offenbarten DNA-Moleküls, aufzufinden.

Bevorzugt umfasst die Probe für das obengenannte Verfahren genomische DNA eines pflanzlichen bzw. Insekten-Organismus. Durch dieses Verfahren wird auf sehr schnelle und effiziente Weise eine große Anzahl von Pflanzen und Insekten auf das Vorhandensein des GlcNAc-α1,3-Fucosyltransferase-Gens untersucht. Auf diese Weise können Pflanzen und Insekten, die dieses Gen nicht aufweisen, selektiert werden bzw. können in solchen Pflanzen und Insekten, die dieses Gen aufweisen, durch ein oben beschriebenes, Verfahren die Exprimierung der GlcNAc-α1,3-Fucosyltransferase unterdrückt bzw. vollständig unterbunden werden, so dass sie anschließend zur Transfektion und Produktion von (humanen) Glykoproteinen eingesetzt werden können.

Genannt werden ebenfalls die DNA-Moleküle, die für eine GlcNAc-α1,3-Fucosyltransferase codieren, die nach den zwei zuletzt genannten Verfahren selektiert und anschließend aus der Probe isoliert wurden. Diese Moleküle können für weitere Untersuchungen eingesetzt werden. Sie können sequenziert und ihrerseits auch als DNA-Sonden zum Auffinden von GlcNAc-α1,3-Fucosyltransferasen verwendet werde. Diese - markierten - DNA-Moleküle werden für Organismen, die den Organismen, aus denen sie isoliert wurden, verwandt sind, effizienter als Sonden fungieren, als die erfindungsgemäßen DNA-Moleküle.

Offenbart wird eine Präparation von klonierter GlcNAc-α1,3-Fucosyltransferase, die Isoformen mit pI-Werten zwischen 6.0 und 9.0, insbesondere zwischen 6.8 und 8.2, aufweist. Der pI-Wert eines Proteins ist derjenige pH-Wert, bei dem seine Nettoladung Null beträgt und ist abhängig von der Aminosäuresequenz, dem Glykosylierungsmuster als auch von der räumlichen Struktur des Proteins. Die GlcNAc-α1,3-Fucosyltransferase umfasst zumindest 7 Isoformen, die einen pI-Wert in diesem Bereich aufweisen. Der Grund für die verschiedenen Isoformen der Transferase sind z.B. unterschiedliche Glykosylierungen sowie limitierte Proteolyse. Versuche zeigten, dass Mungobohnen-Sämlinge von verschiedenen Pflanzen unterschiedliche Verhältnisse der Isozyme aufweisen. Der pI-Wert eines Proteins kann durch isoelektrische Fokussierung, die dem Fachmann bekannt ist, festgestellt werden.

Die Haupt-Isoform des Enzyms weist ein apparentes Molekulargewicht von 54 kDa auf.

Insbesondere weist die Präparation Isoformen mit pI-Werten von 6.8, 7.1 und 7.6 auf.

Beschrieben wird ein Verfahren zur Herstellung von "verpflanzlichten" Kohlenhydrat-Einheiten von menschlichen und anderen Wirbeltier-Glykoproteinen, wobei zu einer Probe, die eine Kohlenhydrat-Einheit bzw. ein Glykoprotein umfasst, Fucose-Einheiten sowie von einem oben beschriebenen DNA-Molekül codierte GlcNAc-α1,3-Fucosyltransferase zugesetzt werden, so dass Fucose in α1,3-Stellung durch die GlcNAc-α1,3-Fucosyltransferase an die Kohlenhydrat-Einheit bzw. das Glykoprotein gebunden wird. Durch das Verfahren zur Klonierung von GlcNAc-α1,3-Fucosyltransferase ist es möglich, große Mengen gereinigtes Enzym herzustellen. Um eine voll aktive Transferase zu erhalten, werden geeignete Reaktionsbedingungen hergestellt. Es hat sich gezeigt, dass die Transferase eine besonders hohe Aktivität bei einem pH-Wert von etwa 7 aufweist, wenn als Puffer 2-(N-Morpho lino)Ethansulfonsäure-HCl verwendet wird. In Gegenwart von bivalenten Kationen, insbesondere Mn2+, wird die Aktivität der rekombinanten Transferase verstärkt. Die Kohlenhydrat-Einheit wird entweder in ungebundener Form oder an ein Protein gebunden zur Probe zugesetzt. Die rekombinante Transferase ist für beide Formen aktiv.

Die Erfindung wird anhand der nachfolgenden Beispiele und der Zeichnungsfiguren, auf die sie selbstverständlich nicht eingeschränkt ist, weiter erläutert werden. Im einzelnen zeigen in der Zeichnung die Fig. 1a und 1b die gemessenen Mengen an Protein und die gemessene Enzymaktivität in den einzelnen Fraktionen des Eluats als Kurven; die Fig. 2 die Abbildung eines Elek trophorese-Gels der GlcNAc-α1,3-Fucosyltransferase; die Fig. 3 das Ergebnis der isoelektrischen Fokussierung und die gemessene Transferase-Aktivität der einzelnen Isoformen; die Fig. 4 die N-terminalen Sequenzen von 4 tryptischen Peptiden 1 - 4 sowie die DNA-Sequenz von drei Primern S1, A2 und A3; die Fig. 5a und 5b die cDNA-Sequenz der α1,3-Fucosyltransferase; die Fig. 6a und 6b die daraus hergeleitete Aminosäuresequenz der α1,3-Fucosyltransferase; die Fig. 7 eine schematische Darstellung der α1,3-Fucosyltransferase sowie die Hydrophobizität der Aminosäuren-Reste; die Fig. 8 einen Vergleich der konservierten Motive verschiedener Fucosyltransferasen; die Fig. 9 einen Vergleich der Fucosyltransferase-Aktivität von mit dem α1,3-Fucosyltransferase-Gen transfektierten Insektenzellen mit einer Negativkontrolle; die Fig. 10a und 10b Strukturen von verschiedenen Akzeptoren der α1,3-Fucosyltransferase; die Fig. 11 und 12 Massenspektren; und die Fig. 13 das Ergebnis einer HPLC.

### Beispiel 1:

### Isolieren der Core-α1,3-Fucosyltransferase

Alle Schritte wurden bei 4°C durchgeführt. Mungobohnen-Sämlinge wurden in einem Mixer homogenisiert, wobei 0.75 Volumen Extraktionspuffer pro kg Bohnen verwendet wurde. Das Homogenisat wurde anschließend durch zwei Lagen Baumwollstoff filtriert und das Filtrat wurde bei 30000xg 40 min zentrifugiert. Der Überstand wurde verworfen und das Pellet wurde mit Lösungspuffer über Nacht bei ständigem Rühren extrahiert. Anschließende Zentrifugation bei 30000xg für 40 min ergab den Triton-Extrakt.

Der Triton-Extrakt wurde wie folgt gereinigt:
Schritt 1: Der Triton-Extrakt wurde auf einen mikrogranulären Diäthylaminoäthyl-Zellulose-Anionenaustauscher DE52 Zellulose-Säule (5x28 cm, Fa. Whatman), der zuvor mit Puffer A kalibriert wurde, aufgetragen. Die nicht gebundene Fraktion wurde in Schritt 2 weiterbehandelt.
Schritt 2: Die Probe wurde auf eine mit Puffer A kalibrierte Affi-Gel Blue Säule (2.5x32) Säule aufgetragen. Nach Waschen der Säule mit diesem Puffer wurde adsorbiertes Protein mit Puffer A umfassend 0.5 M NaCl eluiert.
Schritt 3: Nach Dialyse des Eluats aus Schritt 2 gegen Puffer B wurde es auf eine, mit demselben Puffer kalibrierte, S-Sepharose-Säule aufgetragen. Gebundenes Protein wurde mit einem linearen Gradienten von 0 bis 0.5 M NaCl in Puffer B eluiert. Fraktionen mit GlcNAc-α1,3-Fucosyltranferase wurden gepoolt und gegen Puffer C dialysiert.
Schritt 4: Die dialysierte Probe wurde auf eine mit Puffer C kalibrierte GnGn-Sepharose-Säule aufgetragen. Das gebundene Protein wurde mit Puffer C umfassend 1 M NaCl anstelle von MnCl₂ eluiert.
Schritt 5: Das Enzym wurde anschließend gegen Puffer D dialysiert und auf eine GDP-Hexanolamin-Sepharose-Säule aufgebracht. Nach Waschen der Säule mit Puffer D wurde die Trans ferase durch Ersetzen von MgCl₂ und NaCl mit 0.5 mM GDP eluiert. Aktive Fraktionen wurden gepoolt, gegen 20 mM Tris-HCl Puffer, pH 7.3, dialysiert und lyophilisiert.

Die enzymatische Aktivität der GlcNAc α1,3 Fucosyltransferase wurde durch Verwendung von GnGn-Peptid und GDP-L-[U-¹⁴C] Fucose bei Substratkonzentrationen von jeweils 0.5 und 0.25, in Gegenwart von 2-(N-Morpholin)Ethansulfonsäure-HCl Puffer, Triton X-100, MnCl₂, GlcNAc und AMP bestimmt (gemäß Staudacher et al. 1998 Glycoconjugate J. 15, 355-360; Staudacher et al. 1991 Eur.J.Biochem. 199, 745-751).

Proteinkonzentrationen wurden mit Hilfe der Bichinchoninsäure Methode (Pierce) oder, bei den letzten Schritten der Enzymreinigung, mittels Aminosäurenanalyse bestimmt (Altmann 1992 Anal.Biochem. 204, 215-219).

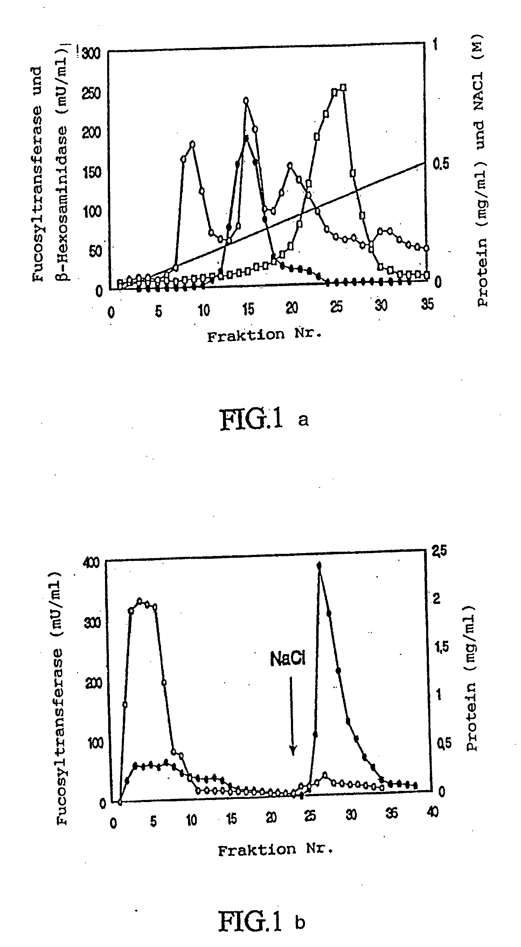

In Fig. 1a und 1b sind die gemessenen Mengen an Protein und die gemessene Enzymaktivität in den einzelnen Fraktionen des Eluats als Kurven dargestellt. Fig. 1a zeigt die oben beschriebene Trennung auf der S-Sepharose Säule, Fig. 1b die Trennung auf der GnGn-Sepharose-Säule, wobei der Kreis Protein, der schwarze, volle Kreis GlcNAc-α1,3-Fucosyltransferase und das Viereck N-Acetyl-β-Glucosaminidase darstellt. Ein U ist definiert als die Menge Enzym, die 1 µmol Fucose auf einen Akzeptor pro Minute tranferiert.

Tabelle 1 zeigt die einzelnen Schritte der Transferase-Reinigung.

**Tabelle 1**

| Reinigungsschritt | Ges. Prot. | Ges. Aktiv. | Spez. Aktiv. | Reinigungs Faktor | Ausbeute |
|---|---|---|---|---|---|
| | *mg* | *mU* | *mU*/*mg* | *-fach* | *%* |
| Triton X-100 Extrakt | 91500 | 4846 | 0.05 | 1 | 100 |
| DE52 | 43700 | 4750 | | 0.10 | |
| 2 | 98.0 | | | | |
| Affigel Blue | 180.5 | 4134 | 23 | 460 | 85.3 |
| S-Sepharose | 8.4 | 3251 | 390 | 7800 | 67.1 |
| GnGn-Sepharose | 0.13¹ | 1044 | 8030 | 160000 | 21.5 |
| GDP-Hexanolamin-Sepharose | 0.02¹ | 867 | 43350 | 867000 | 17.9 |

| | | | | | |
|---|---|---|---|---|---|
| ¹ wurde mittels Aminosäure-Analyse ermittelt | | | | | |

Extraktionspuffer:
   0.5 mM Dithiothreit
   1 mM EDTA
   0.5 % Polyvinylpolypyrrolidon
   0.25 M Saccharose
   50 mM Tris-HCl Puffer, pH 7.3
Lösungspuffer:
   0.5 mM Dithiothreit
   1 mM EDTA
   1.5% Triton X-100
   50 mM Tris-HCl, pH 7.3
Puffer A:
   25mM Tris-HCl Puffer, pH 7.3 mit:
   0.1% Triton X-100 und
   0.02% NaN₈
Puffer B:
   25 mM Na-Citrat Puffer, pH 5.3 mit:
   0.1% Triton X-100 und
   0.02% NaN₃
Puffer C:
   25 mM Tris-HCl Puffer, pH 7.3 mit:
   5 mM MnCl₂ und
   0.02% NaN₃
Puffer D:
   25 mM Tris-HCl, pH 7.3 mit:
   10 mM MgCl₂,
   0.1 M NaCl und
   0.02% NaN₃

### Beispiel 2:

### SDS-Page und isoelektrische Fokussierung

Eine SDS-Page wurde in einer Biorad-Mini-Protean-Zelle auf Gelen mit 12.5% Acrylamid und 1% Bisacrylamid durchgeführt. Die Gele wurden entweder mit Coomassie Brilliant Blau R-250 oder Silber gefärbt. Isoelektrische Fokussierung der Fucosyltransferase wurde auf vorgefertigten Gelen mit einem pI-Bereich zwischen 6-9 (Servalyt precotes 6-9, Serva) durchgeführt. Die Gele wurden gemäß dem Protokoll des Herstellers mit Silber gefärbt. Für die zweidimensionale Elektrophorese wurden Banden aus dem Fokussierungsgel ausgeschnitten, mit S-Alkylierungs-Reagentien und SDS behandelt und einer SDS-Page, wie oben beschrieben, unterzogen.

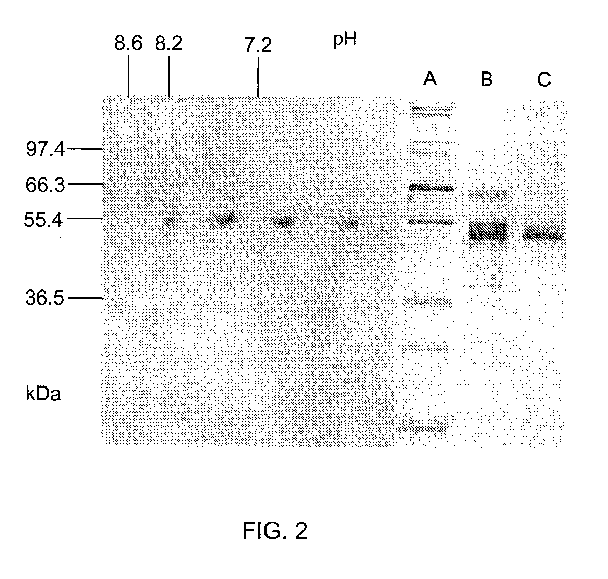

Fig. 2 stellt die Abbildung eines Elektrophorese-Gels der GlcNAc-α1,3-Fucosyltransferase dar, wobei links die zweidimensionale Elektrophorese und rechts die eindimensionale SDS-Page dargestellt sind. Dabei ist die mit A bezeichnete Bahn ein Standard, die mit B bezeichnete Bahn die GlcNAc α1,3-Fucosyltransferase aus der GnGn-Sepharose-Säule und die mit C bezeichnete Bahn "gereinigte" GlcNAc-α1,3-Fucosyltransferase, d.h. die Fraktion der GDP-Hexanolamin-Sepharose-Säule. Die zwei Banden bei 54 und 56 kDa stellen Isoformen der Transferase dar.

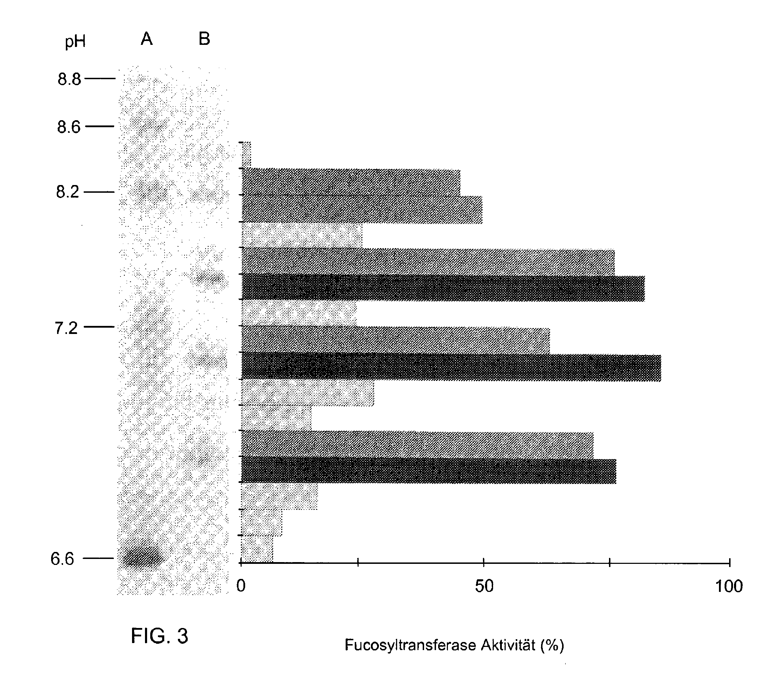

Fig. 3 zeigt das Ergebnis der isoelektrischen Fokussierung. Bahn A wurde mit Silber gefärbt, auf Bahn B wurde Aktivität der Transferase Isoformen getestet. Die Aktivität ist dabei als % Fucose angegeben, die GDP-Fucose auf das Substrat transferiert wurde.

### Beispiel 3:

### Peptidsequenzierung

Für die Sequenzierung des Proteins wurden Banden aus dem mit Coomassie gefärbten SDS-Polyacrylamidgel geschnitten, carboxy amidomethyliert und mit Trypsin gemäß Görg et al. 1988, Electrophoresis, 9, 681-692, gespalten. Die tryptischen Peptide wurden mit der reversen Phase HPLC auf einer 1.0x250 mm Vydac C18 bei 40°C mit einer Durchflussrate von 0.05 ml/min getrennt, wobei ein HP 1100 Apparat (Hewlett-Packard) verwendet wurde. Die isolierten Peptide wurden mit Hewlett-Packard G1005A Protein Sequenzierungs- System gemäß dem Protokoll des Herstellers sequenziert. Weiters wurde die Peptidmischung durch Ingel-Verdauung mit MALDI-TOF MS analysiert (siehe unten).

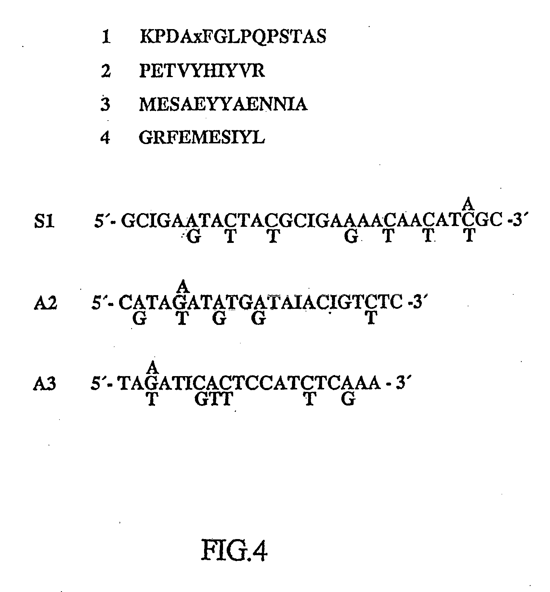

Fig. 4 zeigt die N-terminalen Sequenzen von 4 tryptischen Peptiden 1 - 4 (SEQ ID No: 5-8). Ausgehend von den ersten drei Peptiden wurden Primer_S1, A2 und A3 hergestellt (Sequenz-Identifikationsnummern 9-11).

### Beispiel 4:

### RT-PCR und cDNA Klonieren

Die gesamte RNA wurde aus einem 3 Tage alten Mungo Bohnen Hypokotyl isoliert, wobei das SV Total RNA Isolierungs System von Promega verwendet wurde. Zur Herstellung der Erststrang-cDNA wurde die gesamte RNA 1 h bei 48°C mit AMV reverse Transkriptase und Oligo(dT) Primern inkubiert, wobei das Reverse Transkription System von Promega verwendet wurde.

Die Erststrang-cDNA wurde einer PCR unterzogen, wobei eine Kombination von Sense- und Antisense-Primern verwendet wurde:
Zu 10 µl der reversen Transkription Reaktion wurde folgendes zugesetzt:
   50 µl mit 0.1 µmmol eines jeden Primers, 0.1 mM dNTPs, 2 mM MgCl₂, 10 mM Tris-HCl Puffer, pH 9.0, 50 mM KCl und 0.1 % Triton X-100.

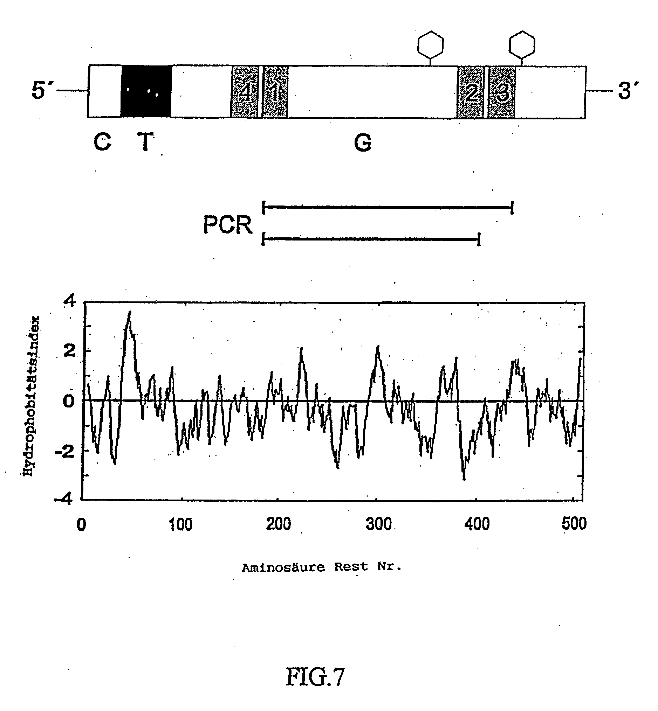

Nach einem ersten Denaturierungsschritt bei 95°C für 2 min, wurden 40 Zyklen von 1 min bei 95°C, 1 min bei 49°C und 2 min bei 72°C durchlaufen. Der letzte Extensionsschritt wurde bei 72°C für 8 min durchgeführt. PCR Produkte wurden in den pCR2.1 Vektor subkloniert, wobei das TA Cloning Kit von Invitrogen verwendet wurde, und sequenziert. Die Produkte dieser PCR waren zwei DNA-Fragmente mit der Länge von 744bp und 780bp, wobei beide DNA-Fragmente dasselbe 5'-Ende aufweisen (s. auch Fig.7).

Ausgehend von diesen zwei DNA-Fragmenten wurden die fehlenden 5' und 3' Regionen der cDNA durch 5'- und 3'- Rapid Amplifikation von cDNA-Enden (RACE) erhalten, wobei der RACE Kit von Gibco-BRL verwendet wurde. Als Antisense-Primer wurde der universelle Amplifikations-Primer des Kits und als Sense-Primer entweder 5'-CTGGAACTGTCCCTGTGGTT-3' (SEQ ID No: 12) oder 5'-AGT-GCACTAGAGGGCCAGAA-3' (SEQ ID No: 13) verwendet. Es wurde auch als Sense Primer der verkürzte Anker-Primer des Kits und als Antisense-Primer 5'-TTCGAGCACCACAATTGGAAAT-3' (SEQ ID No: 14) oder 5'-GAATGCAAAGACGGCACGATGAAT-3' (SEQ ID No: 15) verwendet.

Die PCR wurde mit einer Annealing Temperatur von 55°C und den oben beschriebenen Bedingungen durchgeführt. Die 5' und 3' RACE Produkte wurden in den pCR2.1 Vektor subkloniert und sequenziert: Die Sequenzen der subklonierten Fragmente wurden mittels der Didesoxynukleotid-Methode sequenziert (ABI PRISM Dye Terminator Cycle Sequencing Ready reaction Kit und ABI PRISM 310 Genetic analyser (Perkin Elmer)). T7 und M13 Vorwärts-Primer wurden für die Sequenzierung der in den pCR2.1 Vektor klonierten Produkte verwendet. Beide Stränge der Codierungsregion wurden vom Vienna VBC Genomics-Sequencing Service sequenziert, wobei infrarotmarkierte Primer (IRD700 und IRD800) und ein LI-COR Long Read IR 4200 Sequenzierer (Lincoln, NE) verwendet wurden.

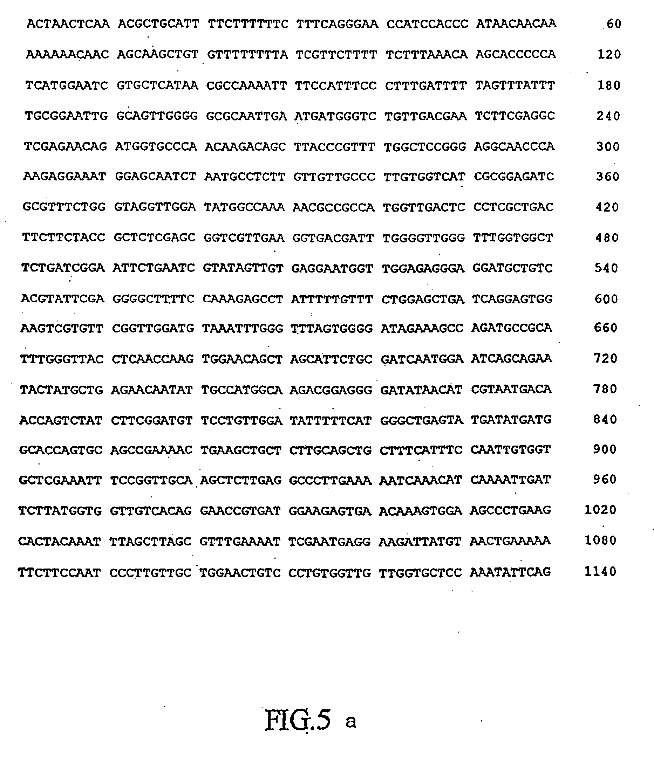

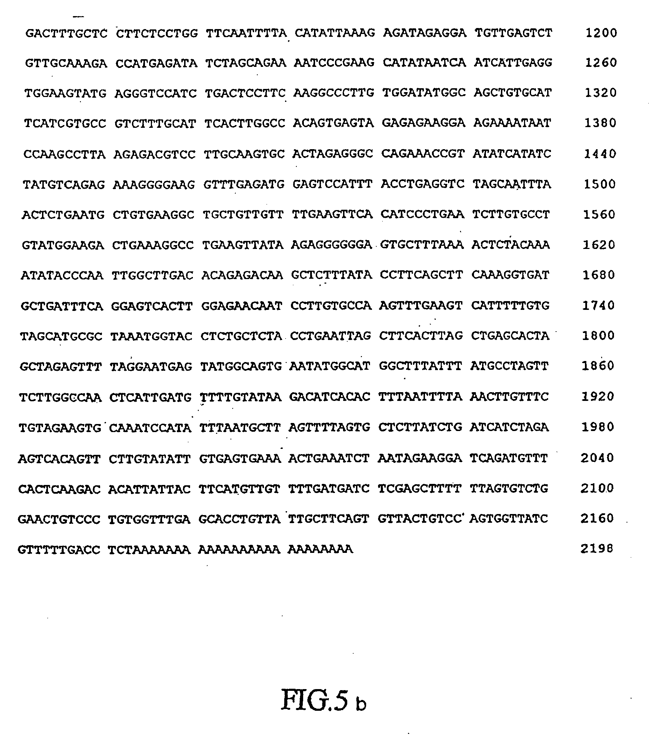

Fig. 5a und 5b zeigen die gesamte cDNA, die eine Größe von 2198 bp und einen offenen Leserahmen von 1530 bp aufweist (SEQ ID No: 1). Der offene Leserahmen (Startcodon bei Basenpaaren 211-213, Stopcodon bei Basenpaar 1740-1743) codiert für ein Protein von 510 Aminosäuren mit einem Molekulargewicht von 56.8 kDA und einem theoretischen pI-Wert von 7.51.

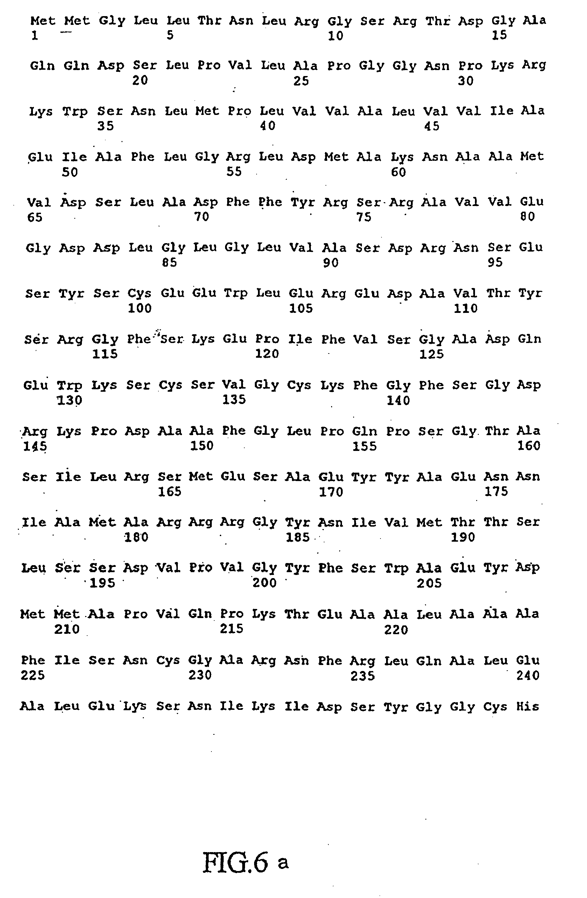

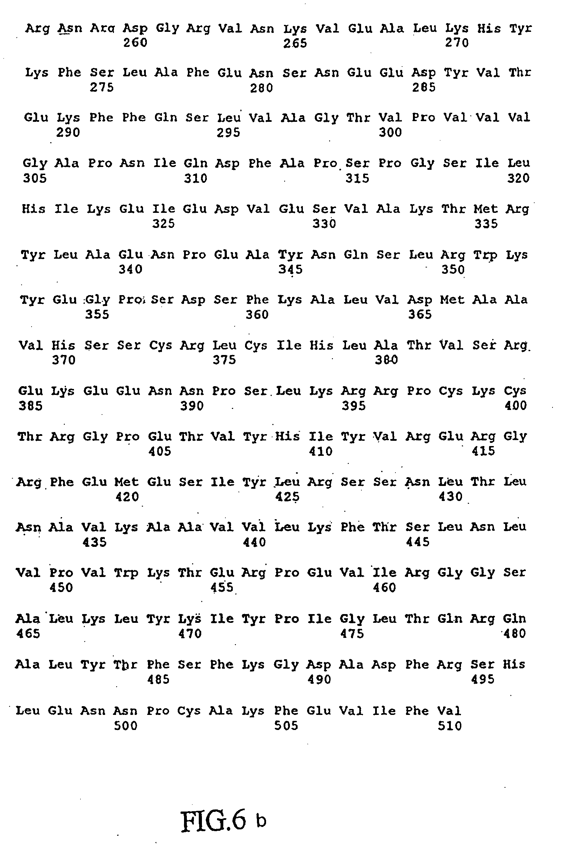

Fig. 6a und 6b zeigen die von der cDNA hergeleitete Aminosäurensequenz der GlcNAc-α1,3 Fucosyltransferase (SEQ ID No: 2). Stellen für die Asparagin gebundene Glykosylierung sind bei Asn346 und Asn429.

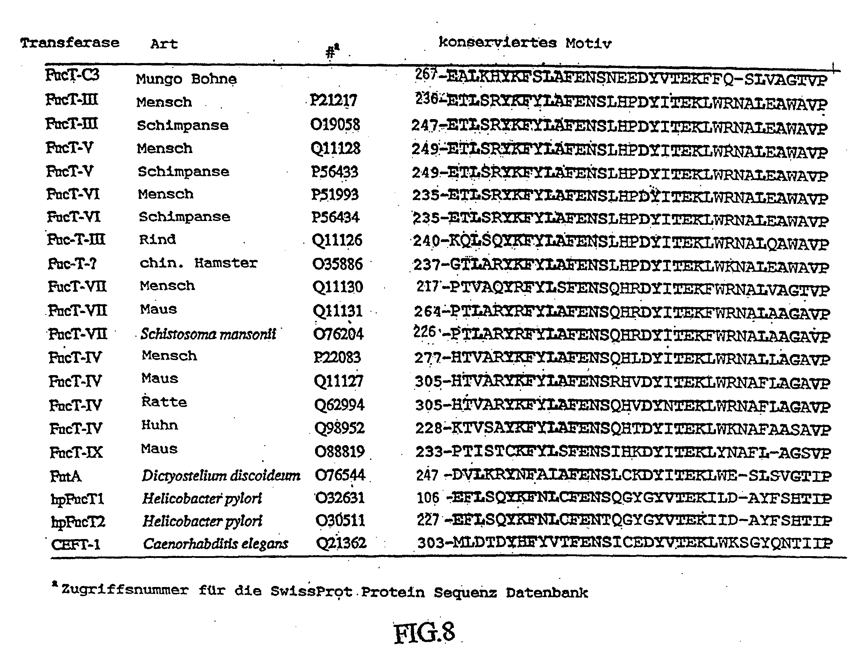

In Fig. 7 ist die schematische GlcNAc-α1,3-Fucosyltransferase-cDNA (oben) und der hergeleitete Hydrophobizitätsindex des codierten Proteins (unten) dargestellt, wobei ein positiver Hydrophobizitätsindex eine erhöhte Hydrophobizität bedeutet. Dazwischen sind die Größen der beiden oben genannten PCR-Produkte in Relation zur kompletten cDNA gezeigt. Der Codierungs-Bereich ist durch den Balken dargestellt, wobei "C" für den postulierten cytoplasmatischen Bereich, T für den postulierten transmembranen Bereich und G für den postulierten Golgi-Lumen katalytischen Bereich der Transferase codiert. Die Analyse der DNA-Sequenz durch "TMpred" (von EMBnet, Schweiz) ergab eine vermutliche Transmembran-Region zwischen Asn36 und Gly54. Der C-terminale Bereich des Enzyms umfasst vermutlich die katalytische Region und sollte folglich in das Lumen des Golgi-Apparats weisen. Demnach scheint diese Transferase ein Transmembranprotein des Typs II zu sein, wie alle bisher analysierten Glykosyltranferasen, die eine Rolle in der Glykoprotein-Biosynthese spielen (Joziasse, 1992 Glycobiology 2, 271-277). Die grauen Bereiche stellen die vier tryptischen Peptide, die Sechsecke die potentiellen N-Glykosylierungs-Stellen dar. Eine via NCBI zugängliche BLASTP Suche aller Datenbanken zeigte eine Ähnlichkeit zwischen der GlcNAc-α1,3 Fucosyltransferase und anderen α1,3/4-Fucosyltransferasen, z.B. der humanen Fucosyltransferase-VI. Mit 18-21% (untersucht von SIM-LALNVIEW, Expase, Schweiz) war die Gesamtähnlichkeit jenseits jeglicher Signifikanz. Dennoch weist ein Sequenzbereich von 35 Aminosäuren (SEQ ID No: 4) eine auffallend hohe Homologie zu anderen α1,3/4 Fucosyltransferasen auf (Fig. 8). Dieser Sequenzbereich befindet sich zwischen Glu267 und Pro301 der SEQ ID No: 2.

### Beispiel 5:

### Expression der rekombinanten GlcNAc-α1,3 Fucosyltransferase in Insektenzellen

Die codierende Region der vermutlichen GlcNAc-α1,3 Fucosyl-transferase samt cytoplasmatischer und transmembraner Region wurde mit dem Vorwärts-Primer 5'-CGGCGGATCCGCAATTGAATGATG-3' (SEQ ID NO: 16) und Rückwärts-Primer 5'- CCGGCTGCAGTACCATTTAGCG-CAT-3' (SEQ ID No: 17) mit dem Expand High Fidelity PCR System von Boehringer Mannheim amplifiziert. Das PCR-Produkt wurde mit PstI und BamHI doppelt verdaut und in Alkalin-Phosphatase behandeltem Baculovirus Transfer-Vektor pVL1393, der zuvor mit PstI und BamHI verdaut wurde, subkloniert. Um eine homologe Rekombination zu gewährleisten, wurde der Transfervektor mit Baculo Gold viraler DNA (PharMingen, San Diego, CA) in Sf9 Insektenzellen in IPL-41 Medium mit Lipofectin cotransfektiert. Nach einer Inkubation von 5 Tagen bei 27°C wurden verschiedene Volumina des Überstandes mit dem rekombinanten Virus zur Infektion der Sf21 Insektenzellen verwendet. Nach einer Inkubation von 4 Tagen bei 27°C in IPL-41 Medium mit 5% FCS wurden die Sf1 Zellen geerntet und 2x mit Phosphat gepufferter Salzlösung gewaschen. Die Zellen wurden in 25 mM Tris HCL Puffer, pH 7.4, mit 2% Triton X-100 resuspendiert und durch Sonifikation auf Eis aufgebrochen.

### Beispiel 6:

### Assay für die GlcNAc α1,3 Fucosyltransferase-Aktivität

Das Homogenat und der Zellüberstand wurden auf GlcNAc α1,3 Fucosyltransferase getestet. Blindproben wurden mit rekombinatem Baculovirus, der für die Tabak-GlcNAc-Transferase I (Strasser et al. 1999, Glycobiology, im Druck) codiert, durchgeführt.

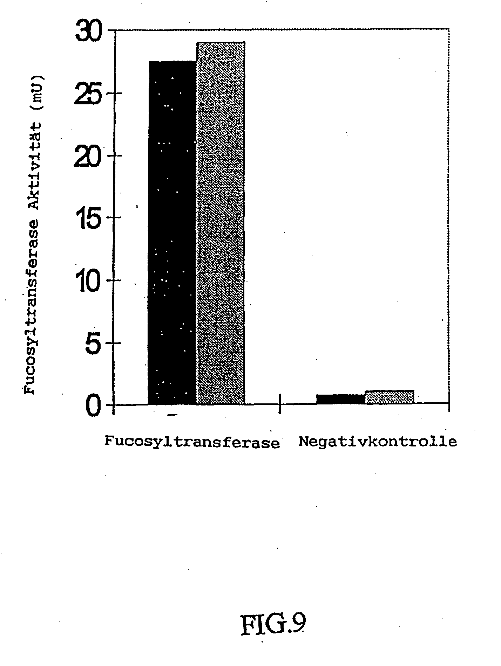

Fig. 9 zeigt die gemessene Enzymaktivität der rekombinanten GlcNAc-α1,3-Fucosyltransferase sowie der Negativkontrolle. Im besten Fall war die Enzymaktivität der cotransfektierten Zellen und deren Überstand 30x höher als die der Negativkontrollen. Diese endogene Aktivität, die in Abwesenheit der rekombinanten Transferase messbar ist, kommt im wesentlichen von der Insekten-α1,6-Fucosyltransferase und nur zu einem geringen Prozentsatz von der GlcNAc-α1,3-Fucosyltransferase. Demnach beträgt die Erhöhung der GlcNAc-α1,3-Fucosyltransferase, die von den rekombinanten Baculoviren stammt, weit über das 100fache.

Das Enzym zeigte eine breite Maximal-Aktivität um einen pH-Wert von 7.0, wenn die Aktivität in 2-(N-Morpholin) Ethansulfonsäure-HCl Puffer gemessen wurde. Wie in Tabelle 2 ersichtlich ist, födert der Zusatz von bivalenten Kationen, insbesondere Mn2+, die Aktivität der rekombinanten Transferase.

**Tabelle 2**

| Zusatzmittel (konz. 10mM) | relative Aktivität (Akzeptor : GnGn-Peptid) |
|---|---|
| | % |
| kein | 21 |
| EDTA | 18 |
| MnCl₂ | 100 |
| CaCl₂ | 82 |
| MgCl₂ | 52 |
| CdCl₂ | 44 |
| CoCl₂ | 35 |
| CuCl₂ | 3 |
| NiC₂ | 24 |
| ZnCl₂ | 0.6 |

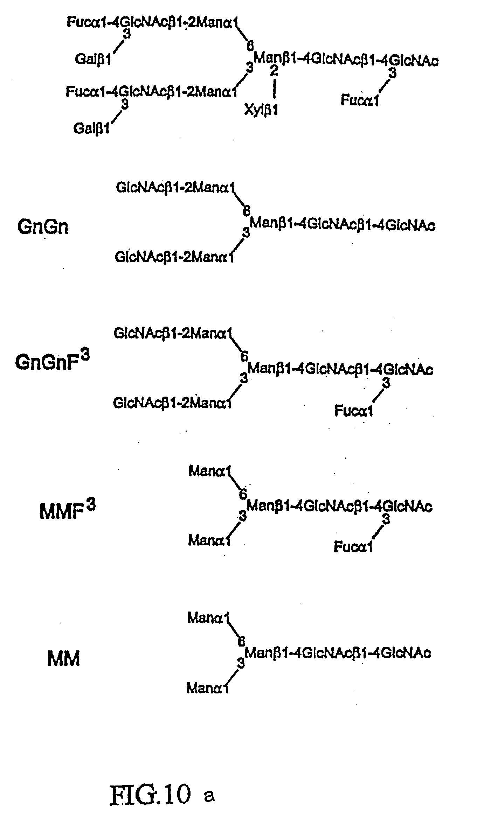

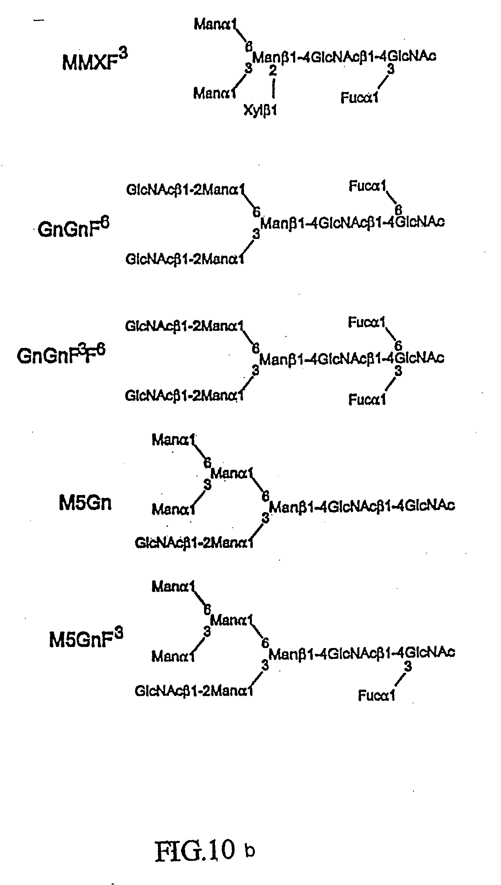

Tabelle 3 zeigt, dass unter den eingesetzten Akzeptoren das GnGn- Peptid die höchsten Inkorporationsraten bei Standard Versuchsbedingungen zeigt, knapp gefolgt vom GnGnF⁶-Peptid und M5Gn-Asn. Kein Transfer konnte auf das MM-Peptid festgestellt werden, welches das reduzierende GlcNAc-Ende an der 3-gebundenen Mannose nicht aufweist. Diese Struktur scheint für die Core-Fucosyltransferase notwendig zu sein. Die rekombinante Transferase war weiters inaktiv hinsichtlich die gebräuchlichen Akzeptoren, die für die Bestimmung der Blutgruppen α1,3/4-Fucosyltransferasen, die die Fucose auf GlcNAc an den nicht reduzierenden Enden von Oligosacchariden transferieren. Die scheinbaren Kₘ-Werte für die Akzeptor-Substrate GnGn-Peptid, GnGnF⁶-Peptid, M5Gn-Asn und für das Donor Substrat GDP-Fucose wurden auf 0.19, 0.13, 0.23 bzw. 0.11 geschätzt. Die Strukturen der Moleküle sind in Fig. 10a und 10b dargestellt.

**Tabelle 3**

| Akzeptor-Substrat | rel. Aktivität | Kₘ-Wert |
|---|---|---|
| | % | mM |
| GnGn-Peptid | 100 | 0.19 |
| GnGnF⁶-Peptid | 87 | 0.13 |
| M5Gn-Asn | 71 | 0.23 |
| MM-Peptid | 0 | |
| Galβ1-4GlcNAc | 0 | |
| Galβ1-3GlcNAc | 0 | |
| Galβ1-3GlcNAcβ1-3Galβ1-4Glc | 0 | |

### Beispiel 7:

### Massenspektrometrie des Fucosyl-Transferase Produkts

Dabsyliertes GnGn-Hexapeptid (2nmol) wurde mit dem Insekten zellen-Homogenat umfassend die rekombinante GlcNAc-α1,3 Fucosyl-transferase (0.08 mU) in Gegenwart von nicht radioaktiver GDP-L-Fucose (10 nmol), 2-(N-Morpholin)Ethansulfonsäure-HCL Puffer, Triton X-100, MnCl₂, GlcNAc und AMP inkubiert. Eine Negativkontrolle wurde mit einem Homogenat der für die Blindproben infizierten Insektenzellen durchgeführt. Die Proben wurden für 16 h bei 37°C inkubiert und mittels MALDI TOF Massenspektrometrie analysiert.

Massenspektrometrie wurde auf einem DYNAMO (Therrmo BioAnalysis, Santa Fe, NM) durchgeführt, einem MALDI-TOF MS, das zur dynamischen Extraktion fähig ist (Synonym für verspätete Extraktion). Es wurden zwei Arten von Probenmatrix-Präparationen eingesetzt: Peptide und dabsylierte Glykopeptide wurden in 5% Ameisensäure gelöst und Aliquote wurden auf das Ziel aufgetragen, Luft getrocknet und mit 1% α-Cyano-4-Hydroxyzimtsäure bedeckt. Pyridylaminierte Glykane, reduzierende Oligosaccharide und nicht derivatisierte Glykopeptide wurden mit Wasser verdünnt, auf das Ziel aufgetragen und Luft getrocknet. Nach Zusatz von 2% 2,5-Dihydroxybenzoesäure wurden die Proben sofort durch Anlegen eines Vakuums getrocknet.

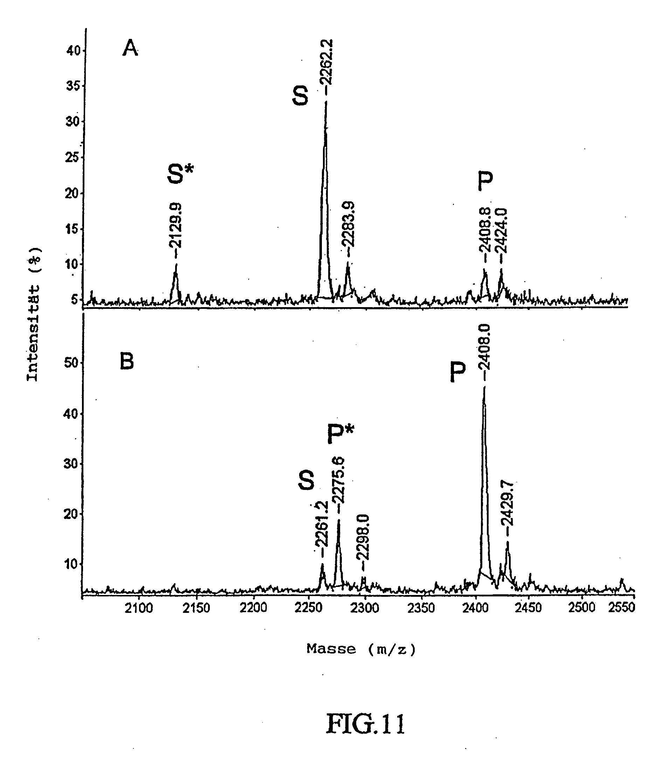

Fig. 11 zeigt das Massenspektrum dieser Proben, wobei A die Negativkontrolle darstellt: Der Hauptpeak (S) zeigt das Dabsyl-Val-Gly-Glu-(GlcNAc₄Man₃)Asn-Arg-Thr Substrat, wobei der errechnete [M+H]⁺ Wert 2262.3 beträgt. Dieses Substrat scheint auch als Natrium Additionsprodukt und als kleineres Ion, das durch Fragmentation der Azo-Funktion der Dabsyl-Gruppe entstanden ist, bei (S*) auf. Eine geringe Produktmenge (P, [M+H]⁺ = 2408.4) ist auf die endogene α1,6-Fucosyltransferase zurückzuführen. Der Peak bei m/z = 2424.0 zeigt die unvollständige Degalactosylierung des Substrats. Das Massenspektrum B zeigt die Probe mit rekombinanter α1,3-Fucosyltransferase. Der Hauptpeak (P) stellt das fucosylierte Produkt dar, (P*) dessen fragmentiertes Ion.

Zusätzlich wurden Aliquote beider Proben miteinander vermischt, um ähnliche Konzentratinen von Substrat und Produkt zu erhalten (Probe A). Diese Mischung wurde mit 0.1 M Ammoniumacetat, pH 4.0, umfassend 10 µU N-Glykosidase A (Probe B) oder mit 50 mM Tris/HCl, pH 8.5, umfassend 100 µU (1 U hydrolysiert 1 µmol Substrat pro min) N-Glykosidase F (Probe C) verdünnt. Nach 2 und 20h wurden kleine Aliquote dieser Mischungen entnommen und mittels MALDI-TOF MS analysiert.

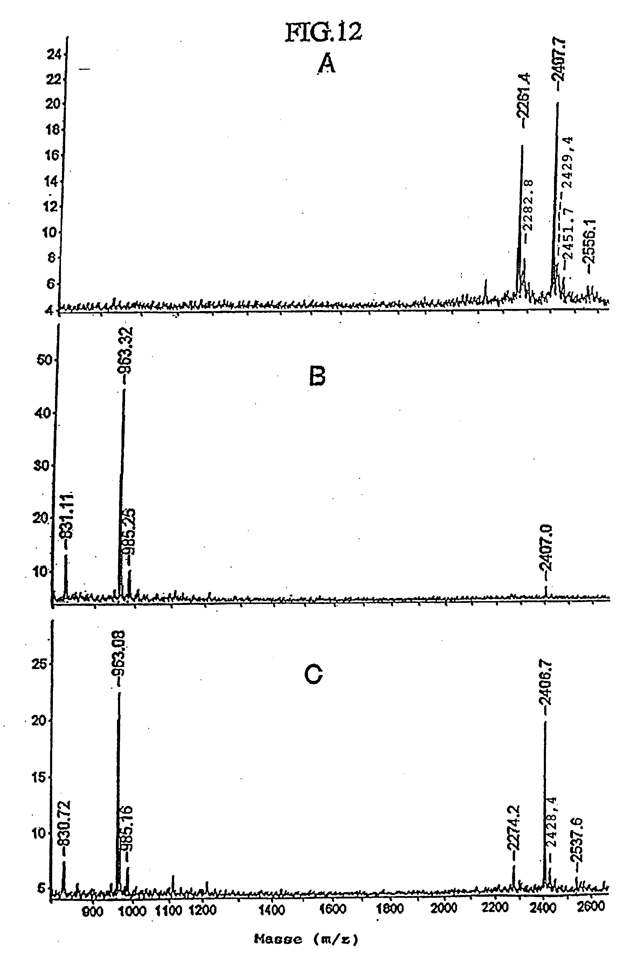

In Fig. 12 sind die drei Massenspektren der Proben A, B und C dargestellt. Die unverdaute Probe A zeigt zwei Hauptpeaks: Das Substrat bei 2261.4 m/z und das fucosylierte Produkt bei 2407.7 m/z. Die mittlere Kurve zeigt das Massenspektrum der Probe B, mit N-Glycosidase A behandelt, die beide Glykopeptide hydrolysiert. Der Peak bei 963.32 stellt das deglycosylierte Produkt dar. Die untere Kurve zeigt das Massenspektrum der Probe C. Die N-Glykosidase F ist nicht in der Lage, α1,3 fucosylierte Substrate zu hydrolysieren, so dass das Spektrum den Peak bei 2406.7 m/z des fucosylierten Produkts aufweist, während der Peak des hydrolysierten Substrats bei 963.08 m/z aufscheint.

### Beispiel 8:

### HPLC Analyse des pyridylaminierten Fucosyltransferase-Produkts

Die beiden oben beschriebenen Proben (fucosyliertes Produkt und Negativkontrolle) wurden mit N-Glykosidase A verdaut. Die erhaltenen Oligosaccharide wurden pyridylaminiert und mittels reverse Phase HPLC analysiert (Wilson et al. 1998 Glycobiology 8, 651-661; Kubelka et al. 1994 Arch.Biochem.Biophys.308, 148-157; Hase et al. 1984 J.Biochem. 95, 197-203).

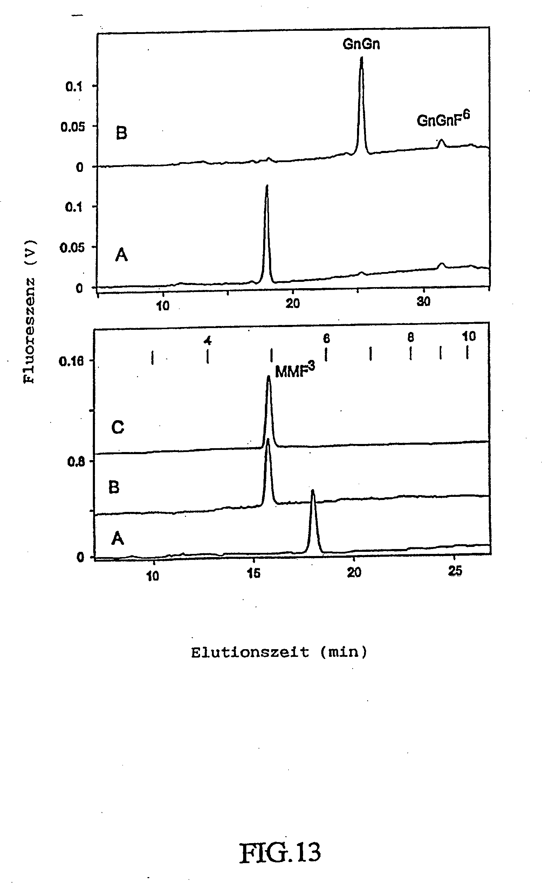

In Fig. 13 stellt im oberen Diagramm B die Negativkontrolle dar, wobei zusätzlich zum Restsubstrat (GnGn-Peptid) α1,6 fucosyliertes Produkt zu sehen ist. A weist einen Peak bei wesentlich geringerer Retentionszeit auf, was für an den reduzierenden GlcNAc-α1,3-gebundene Fucose spezifisch ist.

Im unteren Diagramm wurde das isolierte Transferase-Produkt vor (Kurve A) und nach Verdauung durch N-Acetyl-β-Glukosaminidase (Kurve B) mit MMF³ aus Honigbiene Phospholipase A₂ (Kurve C) verglichen.

## Patentansprüche

1. Verfahren zur Herstellung rekombinanter Glykoproteine, umfassend die Produktion eines rekombinanten Glykoproteins in Pflanzen oder Pflanzenzellen, deren GlcNAc-α1,3-Fucosyltransferaseproduktion unterdrückt oder vollständig unterbunden ist und deren endogene GlcNAc-α1,3-Fucosyltransferase-Aktivität unter 50% der in natürlichen Pflanzen bzw. Pflanzenzellen vorkommenden GlcNAc-α1,3-Fucosyltransferase-Aktivität beträgt, und deren GlcNAc-α-1,3-Fukosyltransferase durch ein DNA-Molekül, das eine Sequenz gemäß der SEQ ID No. 1 mit einem offenen Leserahmen von Basenpaar 211 bis Basenpaar 1740 umfasst oder zumindest 50 % Homologie zur oben genannten Sequenz aufweist oder unter stringenten Bedingungen mit der oben genannte Sequenz hybridisiert oder eine Sequenz umfasst, die in Folge des genetischen Codes zur oben genannte DNA-Sequenz degeneriert ist, codiert ist.

2. Verfahren nach Anspruch 1, **dadurch gekennzeichnet, dass** die rekombinanten Glykoproteine humane Proteine, insbesondere Proteine zur medizinischen Verwendung, sind.

3. Verfahren nach Anspruch 1 oder 2, **dadurch gekennzeichnet, dass** die endogene GlcNAc-α1,3-Fucosyltransferase-Aktivität unter 20% der in natürlichen Pflanzen bzw. Pflanzenzellen vorkommenden GlcNAc-α1,3-Fucosyltransferase-Aktivität beträgt.

4. Verfahren nach einem der Ansprüche 1 bis 3, **dadurch gekennzeichnet, dass** die endogene GlcNAc-α1,3-Fucosyltransferase-Aktivität 0% der in natürlichen Pflanzen bzw. Pflanzenzellen vorkommenden GlcNAc-α1,3-Fucosyltransferase-Aktivität beträgt.

5. Verfahren nach einem der Ansprüche 1 bis 4, **dadurch gekennzeichnet, dass** die Reduktion der GlcNAc-α1,3-Fucosyltransferase-Aktivität durch antisense Inhibition erfolgt, wobei ein Polynukleotid verwendet wird, das zumindest teilweise komplementär zu der Sequenz des DNA-Moleküls ist.

6. Verfahren nach einem der Ansprüche 1 bis 4, **dadurch gekennzeichnet, dass** die Reduktion der GlcNAc-α1,3-Fucosyltransferase-Aktivität durch knock-out Mutation der Sequenz des DNA-Moleküls erfolgt.

## Claims

1. A method for producing recombinant glycoproteins, comprising producing a recombinant glycoprotein in plants or plant cells the GlcNAc-α1,3-fucosyltransferase production of which has been suppressed or completely prevented and for which its endogenous GlcNAc-α1,3-fucosyltransferase activity is less than 50% of the GlcNAc-α1,3-fucosyltransferase activity present in natural plants or plant cells, and its GlcNAc-α1,3-fucosyltransferase is coded for by a DNA molecule comprising a sequence in accordance with SEQ ID No. 1 with an open reading frame from base pair 211 to base pair 1740 or which has at least 50% homology with the above sequence or which hybridizes under stringent conditions with the above sequence or which comprises a sequence which is degenerate with the above DNA sequence due to the genetic code.

2. The method as claimed in claim 1, **characterized in that** the recombinant glycoproteins are human proteins, in particular proteins for medical use.

3. The method as claimed in claim 1 or claim 2, **characterized in that** the endogenous GlcNAc-α1,3-fucosyltransferase activity is less than 20% of the GlcNAc-α1,3-fucosyltransferase activity present in natural plants or plant cells.

4. The method as claimed in one of claims 1 to 3, **characterized in that** the endogenous GlcNAc-α1,3-fucosyltransferase activity is 0% of the GlcNAc-α1,3-fucosyltransferase activity present in natural plants or plant cells.

5. The method as claimed in one of claims 1 to 4, **characterized in that** the reduction in the GlcNAc-α1,3-fucosyltransferase activity is by antisense inhibition, whereby a polynucleotide is used which is at least partially complementary to the sequence of the DNA molecule.

6. The method as claimed in one of claims 1 to 4, **characterized in that** the reduction in the GlcNAc-α1,3-fucosyltransferase activity is by knock-out mutation of the sequence of the DNA molecule.

## Revendications

1. Procédé de production de glycoprotéines recombinantes, comprenant la production d'une glycoprotéine recombinante dans des plantes ou des cellules de plantes, dont la production de GlcNAc-α1,3-fucosyltransférase est inhibée ou complètement arrêtée et dont l'activité de la GlcNAc-α1,3-fucosyltransférase endogène est inférieure à 50 % de l'activité de la GlcNAc-α1,3-fucosyltransférase présente dans les plantes ou les cellules de plantes naturelles et dont la GlcNAc-α1,3-fucosyltransférase est codée par une molécule d'ADN qui comprend une séquence selon la SEQ ID N° : 1 dotée d'un cadre de lecture ouvert de la paire de bases 211 à la paire de bases 1740 ou présente une homologie d'au moins 50 % par rapport à la séquence mentionnée ci-dessus ou est hybridée dans des conditions stringentes avec la séquence mentionnée ci-dessus ou comprend une séquence qui est dégénérée en raison du code génétique par rapport à la séquence d'ADN mentionnée ci-dessus.

2. Procédé selon la revendication 1, **caractérisé en ce que** les glycoprotéines recombinantes sont des protéines humaines, en particulier, des protéines pour l'utilisation médicale.

3. Procédé selon la revendication 1 ou 2, **caractérisé en ce que** l'activité de la GlcNAc-α1,3-fucosyltransférase endogène est inférieure à 20 % de l'activité de la GlcNAc-α1,3-fucosyltransférase présente dans les plantes ou les cellules de plantes naturelles.

4. Procédé selon l'une des revendications 1 à 3, **caractérisé en ce que** l'activité de la GlcNAc-α1,3-fucosyltransférase endogène est à 0 % de l'activité de la GlcNAc-α1,3-fucosyltransférase présente dans les plantes ou les cellules de plantes naturelles.

5. Procédé selon l'une des revendications 1 à 4, **caractérisé en ce que** la réduction de l'activité de la GlcNAc-α1,3-fucosyltransférase s'effectue par inhibition anti-sens, dans lequel on utilise un polynucléotide qui est au moins partiellement complémentaire de la séquence de la molécule d'ADN.

6. Procédé selon l'une des revendications 1 à 4, **caractérisé en ce que** la réduction de l'activité de la GlcNAc-α1,3-fucosyltransférase s'effectue par une mutation knock-out de la séquence de la molécule d'ADN.


### SEQUENCE LISTING

<110> Altmann Dr., Friedrich
<120> Fucosyltransferase-Gen
<130> R 53926 (divisional of R 46233)
<150> A 270/99
   <151> 1999-02-18
<150> EP 05108012.5
   <151> 2000-02-17
<160> 17
<170> PatentIn Ver. 2.1
<210> 1
   <211> 2198
   <212> DNA
   <213> vigna radiata
<400> 1
   actaactcaa acgctgcatt ttcttttttc tttcagggaa ccatccaccc ataacaacaa 60
   aaaaaacaac agcaagctgt gtttttttta tcgttctttt tctttaaaca agcaccccca 120
   tcatggaatc gtgctcataa cgccaaaatt ttccatttcc ctttgatttt tagtttattt 180
   tgcggaattg gcagttgggg gcgcaattga atgatgggtc tgttgacgaa tcttcgaggc 240
   tcgagaacag atggtgccca acaagacagc ttacccgttt tggctccggg aggcaaccca 300
   aagaggaaat ggagcaatct aatgcctctt gttgttgccc ttgtggtcat cgcggagatc 360
   gcgtttctgg gtaggttgga tatggccaaa aacgccgcca tggttgactc cctcgctgac 420
   ttcttctacc gctctcgagc ggtcgttgaa ggtgacgatt tggggttggg tttggtggct 480
   tctgatcgga attctgaatc gtatagttgt gaggaatggt tggagaggga ggatgctgtc 540
   acgtattcga ggggcttttc caaagagcct atttttgttt ctggagctga tcaggagtgg 600
   aagtcgtgtt cggttggatg taaatttggg tttagtgggg atagaaagcc agatgccgca 660
   tttgggttac ctcaaccaag tggaacagct agcattctgc gatcaatgga atcagcagaa 720
   tactatgctg agaacaatat tgccatggca agacggaggg gatataacat cgtaatgaca 780
   accagtctat cttcggatgt tcctgttgga tatttttcat gggctgagta tgatatgatg 840
   gcaccagtgc agccgaaaac tgaagctgct cttgcagctg ctttcatttc caattgtggt 900
   gctcgaaatt tccggttgca agctcttgag gcccttgaaa aatcaaacat caaaattgat 960
   tcttatggtg gttgtcacag gaaccgtgat ggaagagtga acaaagtgga agccctgaag 1020
   cactacaaat ttagcttagc gtttgaaaat tcgaatgagg aagattatgt aactgaaaaa 1080
   ttcttccaat cccttgttgc tggaactgtc cctgtggttg ttggtgctcc aaatattcag 1140
   gactttgctc cttctcctgg ttcaatttta catattaaag agatagagga tgttgagtct 1200
   gttgcaaaga ccatgagata tctagcagaa aatcccgaag catataatca atcattgagg 1260
   tggaagtatg agggtccatc tgactccttc aaggcccttg tggatatggc agctgtgcat 1320
   tcatcgtgcc gtctttgcat tcacttggcc acagtgagta gagagaagga agaaaataat 1380
   ccaagcctta agagacgtcc ttgcaagtgc actagagggc cagaaaccgt atatcatatc 1440
   tatgtcagag aaaggggaag gtttgagatg gagtccattt acctgaggtc tagcaattta 1500
   actctgaatg ctgtgaaggc tgctgttgtt ttgaagttca catccctgaa tcttgtgcct 1560
   gtatggaaga ctgaaaggcc tgaagttata agagggggga gtgctttaaa actctacaaa 1620
   atatacccaa ttggcttgac acagagacaa gctctttata ccttcagctt caaaggtgat 1680
   gctgatttca ggagtcactt ggagaacaat ccttgtgcca agtttgaagt catttttgtg 1740
   tagcatgcgc taaatggtac ctctgctcta cctgaattag cttcacttag ctgagcacta 1800
   gctagagttt taggaatgag tatggcagtg aatatggcat ggctttattt atgcctagtt 1860
   tcttggccaa ctcattgatg ttttgtataa gacatcacac tttaatttta aacttgtttc 1920
   tgtagaagtg caaatccata tttaatgctt agttttagtg ctcttatctg atcatctaga 1980
   agtcacagtt cttgtatatt gtgagtgaaa actgaaatct aatagaagga tcagatgttt 2040
   cactcaagac acattattac ttcatgttgt tttgatgatc tcgagctttt ttagtgtctg 2100
   gaactgtccc tgtggtttga gcacctgtta ttgcttcagt gttactgtcc agtggttatc 2160
   gtttttgacc tctaaaaaaa aaaaaaaaaa aaaaaaaa                        2198
<210> 2
   <211> 510
   <212> PRT
   <213> vigna radiata
<400> 2
   Met Met Gly Leu Leu Thr Asn Leu Arg Gly Ser Arg Thr Asp Gly Ala
   1               5                   10                  15
   Gln Gln Asp Ser Leu Pro Val Leu Ala Pro Gly Gly Asn Pro Lys Arg
               20                  25                  30
   Lys Trp Ser Asn Leu Met Pro Leu Val Val Ala Leu Val Val Ile Ala
           35                  40                  45
   Glu Ile Ala Phe Leu Gly Arg Leu Asp Met Ala Lys Asn Ala Ala Met
       50                  55                  60
   Val Asp Ser Leu Ala Asp Phe Phe Tyr Arg Ser Arg Ala Val Val Glu
   65                  70                  75                  80
   Gly Asp Asp Leu Gly Leu Gly Leu Val Ala Ser Asp Arg Asn Ser Glu
                   85                  90                  95
   Ser Tyr Ser Cys Glu Glu Trp Leu Glu Arg Glu Asp Ala Val Thr Tyr
               100                 105                 110
   Ser Arg Gly Phe Ser Lys Glu Pro Ile Phe Val Ser Gly Ala Asp Gln
           115                 120                 125
   Glu Trp Lys Ser Cys Ser Val Gly Cys Lys Phe Gly Phe Ser Gly Asp
       130                 135                 140
   Arg Lys Pro Asp Ala Ala Phe Gly Leu Pro Gln Pro Ser Gly Thr Ala
   145                 150                 155                 160
   Ser Ile Leu Arg Ser Met Glu Ser Ala Glu Tyr Tyr Ala Glu Asn Asn
                   165                 170                 175
   Ile Ala Met Ala Arg Arg Arg Gly Tyr Asn Ile Val Met Thr Thr Ser
               180                 185                 190
   Leu Ser Ser Asp Val Pro Val Gly Tyr Phe Ser Trp Ala Glu Tyr Asp
           195                 200                 205
   Met Met Ala Pro Val Gln Pro Lys Thr Glu Ala Ala Leu Ala Ala Ala
       210                 215                 220
   Phe Ile Ser Asn Cys Gly Ala Arg Asn Phe Arg Leu Gln Ala Leu Glu
   225                 230                 235                 240
   Ala Leu Glu Lys Ser Asn Ile Lys Ile Asp Ser Tyr Gly Gly Cys His
                   245                 250                 255
   Arg Asn Arg Asp Gly Arg Val Asn Lys Val Glu Ala Leu Lys His Tyr
               260                 265                 270
   Lys Phe Ser Leu Ala Phe Glu Asn Ser Asn Glu Glu Asp Tyr Val Thr
           275                 280                 285
   Glu Lys Phe Phe Gln Ser Leu Val Ala Gly Thr Val Pro Val Val Val
       290                 295                 300
   Gly Ala Pro Asn Ile Gln Asp Phe Ala Pro Ser Pro Gly Ser Ile Leu
   305                 310                 315                 320
   His Ile Lys Glu Ile Glu Asp Val Glu Ser Val Ala Lys Thr Met Arg
                   325                 330                 335
   Tyr Leu Ala Glu Asn Pro Glu Ala Tyr Asn Gln Ser Leu Arg Trp Lys
               340                 345                 350
   Tyr Glu Gly Pro Ser Asp Ser Phe Lys Ala Leu Val Asp Met Ala Ala
           355                 360                 365
   Val His Ser Ser Cys Arg Leu Cys Ile His Leu Ala Thr Val Ser Arg
       370                 375                 380
   Glu Lys Glu Glu Asn Asn Pro Ser Leu Lys Arg Arg Pro Cys Lys Cys
   385                 390                 395                 400
   Thr Arg Gly Pro Glu Thr Val Tyr His Ile Tyr Val Arg Glu Arg Gly
                   405                 410                 415
   Arg Phe Glu Met Glu Ser Ile Tyr Leu Arg Ser Ser Asn Leu Thr Leu
               420                 425                 430
   Asn Ala Val Lys Ala Ala Val Val Leu Lys Phe Thr Ser Leu Asn Leu
           435                 440                 445
   Val Pro Val Trp Lys Thr Glu Arg Pro Glu Val Ile Arg Gly Gly Ser
       450                 455                 460
   Ala Leu Lys Leu Tyr Lys Ile Tyr Pro Ile Gly Leu Thr Gln Arg Gln
   465                 470                 475                 480
   Ala Leu Tyr Thr Phe Ser Phe Lys Gly Asp Ala Asp Phe Arg Ser His
                   485                 490                 495
   Leu Glu Asn Asn Pro Cys Ala Lys Phe Glu Val Ile Phe Val
               500                 505                 510
<210> 3
   <211> 105
   <212> DNA
   <213> Artificial sequence
<220>
   <223> Description of Artificial Sequence:cDNA
<400> 3
   gaagccctga agcactacaa atttagctta gcgtttgaaa attcgaatga ggaagattat 60
   gtaactgaaa aattcttcca atcccttgtt gctggaactg tccct                 105
<210> 4
   <211> 35
   <212> PRT
   <213> Artificial Sequence
<220>
   <223> Description of Artificial Sequence:peptide fragment
<400> 4
   Glu Ala Leu Lys His Tyr Lys Phe Ser Leu Ala Phe Glu Asn Ser Asn
   1               5                   10                  15
   Glu Glu Asp Tyr Val Thr Glu Lys Phe Phe Gln Ser Leu Val Ala Gly
               20                  25                  30
   Thr Val Pro
           35
<210> 5
   <211> 15
   <212> PRT
   <213> Artificial sequence
<220>
   <223> Description of Artificial sequence:peptide fragment
<220>
   <221> misc_feature
   <222> (5)
   <223> unsure
<400> 5
   Lys Pro Asp Ala Xaa Phe Gly Leu Pro Gln Pro Ser Thr Ala Ser
   1               5                   10                  15
<210> 6
   <211> 10
   <212> PRT
   <213> Artificial Sequence
<220>
   <223> Description of Artificial Sequence:peptide fragment
<400> 6
   Pro Glu Thr Val Tyr His Ile Tyr Val Arg
   1               5                   10
<210> 7
   <211> 13
   <212> PRT
   <213> Artificial sequence
<220>
   <223> Description of Artificial sequence:peptide fragment
<400> 7
   Met Glu Ser Ala Glu Tyr Tyr Ala Glu Asn Asn Ile Ala
   1               5                   10
<210> 8
   <211> 10
   <212> PRT
   <213> Artificial sequence
<220>
   <223> Description of Artificial Sequence:peptide fragment
<400> 8
   Gly Arg Phe Glu Met Glu Ser Ile Tyr Leu
   1               5                   10
<210> 9
   <211> 29
   <212> DNA
   <213> Artificial sequence
<220>
   <223> Description of Artificial Sequence:universal primer
<220>
   <221> misc_feature
   <222> (3),(15)
   <223> n is a, c, g or t
<400> 9
   gcngartayt aygcngaraa yaayathgc 29
<210> 10
   <211> 22
   <212> DNA
   <213> Artificial Sequence
<220>
   <223> Description of Artificial Sequence:universal primer
<220>
   <221> misc_feature
   <222> (14),(17)
   <223> n is a, c, g or t
<400> 10
   crtadatrtg rtanacngty tc 22
<210> 11
   <211> 20
   <212> DNA
   <213> Artificial Sequence
<220>
   <223> Description of Artificial Sequence:universal primer
<220>
   <221> misc_feature
   <222> (6)
   <223> n is a, c, g or t
<400> 11
   tadatnswyt ccatytcraa 20
<210> 12
   <211> 20
   <212> DNA
   <213> Artificial Sequence
<220>
   <223> Description of Artificial sequence:primer
<400> 12
   ctggaactgt ccctgtggtt 20
<210> 13
   <211> 20
   <212> DNA
   <213> Artificial sequence
<220>
   <223> Description of Artificial sequence:primer
<400> 13
   agtgcactag agggccagaa 20
<210> 14
   <211> 22
   <212> DNA
   <213> Artificial sequence
<220>
   <223> Description of Artificial sequence:primer
<400> 14
   ttcgagcacc acaattggaa at 22
<210> 15
   <211> 24
   <212> DNA
   <213> Artificial sequence
<220>
   <223> Description of Artificial sequence:primer
<400> 15
   gaatgcaaag acggcacgat gaat 24
<210> 16
   <211> 24
   <212> DNA
   <213> Artificial sequence
<220>
   <223> Description of Artificial sequence:primer
<400> 16
   cggcggatcc gcaattgaat gatg 24
<210> 17
   <211> 25
   <212> DNA
   <213> Artificial sequence
<220>
   <223> Description of Artificial Sequence:primer
<400> 17
   ccggctgcag taccatttag cgcat 25